# EUROPEAN PATENT SPECIFICATION

(11) **EP 1 725 668 B1**
(45) Date of publication and mention of the grant of the patent: **15.12.2010**
(21) Application number: 05718425.1
(22) Date of filing: 18.03.2005
(51) Int. Cl.: C12N 15/82, C07K 14/415, C12N 5/04

(54) **GENETICALLY MODIFIED PLANTS AND THEIR APPLICATIONS IN PHYTOREMEDIATION.**
GENMODIFIZIERTE PFLANZEN UND DEREN VERWENDUNG IN PHYTOREMEDIATION
PLANTES GENETIQUEMENT MODIFIEES ET LEURS APPLICATIONS EN PHYTOREMEDIATION

(30) Priority: 18.03.2004 WO PCT/IB2004/001271
(43) Date of publication of application: 29.11.2006
(73) Proprietor: Commissariat à l'Énergie Atomique et aux Énergies Alternatives, 75015 Paris (FR)
(72) Inventor: RICHAUD, Pierre, F-84120 La Bastidonne (FR); VERRET, Frédéric, PL3 4HD Plymouth (GB); GRAVOT, Antoine, F-35590 L'Hermitage (FR); AUROY, Pascaline, F-84360 Lauris (FR); VAVASSEUR, Alain, F-13090 Aix En Provence (FR)
(74) Representative: Marcadé, Véronique
(86) International application number: PCT/IB2005/000961
(87) International publication number: WO 2005/090583

(56) References cited:
- WO-A-02/081707
- GRAVOT A ET AL: "AtHMA3, a plant P1B-ATPase, functions as a Cd/Pb transporter in yeast" FEBS LETTERS, ELSEVIER SCIENCE PUBLISHERS, AMSTERDAM, NL, vol. 561, no. 1-3, 12 March 2004 (2004-03-12), pages 22-28, XP004495447 ISSN: 0014-5793
- MILLS REBECCA F ET AL: "Functional expression of AtHMA4, a P1B-type ATPase of the Zn/Co/Cd/Pb subclass." THE PLANT JOURNAL: FOR CELL AND MOLECULAR BIOLOGY. ENGLAND JUL 2003, vol. 35, no. 2, July 2003 (2003-07), pages 164-176, XP002287816 ISSN: 0960-7412 cited in the application
- SONG WON-YONG ET AL: "Engineering tolerance and accumulation of lead and cadmium in transgenic plants." NATURE BIOTECHNOLOGY, vol. 21, no. 8, August 2003 (2003-08), pages 914-919, XP002287817 ISSN: 1087-0156 (ISSN print)
- KARENLAMPI S ET AL: "Genetic engineering in the improvement of plants for phytoremediation of metal polluted soils" ENVIRON POLLUT;ENVIRONMENTAL POLLUTION 2000 ELSEVIER SCIENCE LTD, ENGL, vol. 107, no. 2, 2000, pages 225-231, XP002287818
- SINGH O V ET AL: "Phytoremediation: an overview of metallic ion decontamination from soil" APPLIED MICROBIOLOGY AND BIOTECHNOLOGY, SPRINGER VERLAG, BERLIN, DE, vol. 61, 26 February 2003 (2003-02-26), pages 405-412, XP002982032 ISSN: 0175-7598
- WILLIAMS L E ET AL: "Emerging mechanisms for heavy metal transport in plants" BIOCHIMICA ET BIOPHYSICA ACTA. BIOMEMBRANES, AMSTERDAM, NL, vol. 1465, no. 1-2, 1 May 2000 (2000-05-01), pages 104-126, XP004273203 ISSN: 0005-2736 cited in the application

## Description

The instant invention relates generally to the field of phytoremediation. More particularly the invention relates to genetically modified plants able to accumulate heavy metals in shoots and to methods of removing and possibly recovering said heavy metals, using said genetically modified plants.

Numerous ecological and health problems are associated with environmental contamination. Heavy metals contamination of soils is a particular hazard, due to the almost indefinite environmental persistance of metals. While some heavy metals are naturally present in soil, heavy metal contamination is often the result of human activities. Contamination of the environment with heavy metals has increased drastically due to industrialization: mining, smelting, intensive agriculture, sludge dumping, energy and fuel production, electroplating and fuel exhaust contribute to contaminating the soil with heavy metals.

The soils contaminated with heavy metals inhibit normal plant growth and cause contamination of foodstuffs. Many heavy metals are very toxic to human health and carcinogenic at low concentrations. Therefore removal of heavy metals from the environment is crucial.

Currently, the most common methods for dealing with this problem remain removal and burial of the contaminated soil or the isolation of the contaminated area. However, such methods are extremely expensive.

One relatively new option for decontaminating soil is phytoremediation, which uses plants to scavenge organic and inorganic contaminants from the soil.

Phytoremediation may be divided into phytoextraction, rhizofiltration and phytostabilization:
- Phytoextraction is a method using contaminant-accumulating plants to extract said contaminants from soil into the harvestable parts of the plants;
- Rhizofiltration is a method using plant roots to remove contaminants from polluted aqueous streams; and
- Phytostabilization is the stabilization of contaminants such as toxic metals in soils to prevent their entry into ground water, also with plants.

However, usually most plants are unable to grow in soil containing high levels of heavy metals.

Certain plants have been found to accumulate nickel, cobalt, copper, lead, zinc, cadmium and selenium. Accumulators of nickel are by far the most common amongst the hyper accumulators discovered to date. The more rarely absorbed heavy metals include manganese, cadmium and lead, with lead being particularly hard to absorb as well as being very difficult to translocate from plant roots into plant stems after it is absorbed.

Therefore, there is a need for plants being able to grow in soils containing high concentrations of heavy metals. To confer heavy metal resistance to plants, it has been proposed to use the properties of heavy metal-transporting P-type ATPase and more specifically the P_{1B}-ATPases which are known to be heavy metal transporters.

The P_{1B}-ATPases are transporters that use the energy liberated in the exergonic ATP hydrolysis reaction to translocate positively charged substrates across membranes (Inesi, 1985). P_{1B}-ATPases are found in all living organisms, from archaea to humans and are thought to transport soft metal cations. They are referred to as HMAs (Axelsen and Palmgren, 2001; Williams *et al.,* 2000) or CPx-type ATPases (Solioz and Vulpe, 1996; Rensing *et al.,* 1997; Williams *et al.,* 2000).

In the yeast *Saccharomyces cerevisiae,* only 2 P_{1B}-ATPases have been found (Catty *et al.,* 1997), CCC2 and PCA1, both participating in copper transport (Fu *et al.,* 1995; Bassett Jr *et al.,* 1996; Ran *et al.,* 1994).

In *Arabidopsis thaliana,* seven P_{1B}-ATPases were previously identified (Axelsen and Palmgren, 2001) but the recent genome release revealed an eighth gene belonging to the HMAs group (P-type ATPase database; http://biobase.dk/~axe/Patbase.html).

The recent completion of two draft sequences of the rice (*Oryza sativa*) genome shows a similar number of enzymes within this group. Compared with other eukaryotes, which only possess one or two P_{1B}-ATPases, the parallel expansion of the number of HMAs in *Arabidopsis* and *Oryza* suggests that these enzymes play important roles in the overall movements of metals in plants.

In both species, these eight proteins have been classified in 6 clusters, based on full-length sequence alignments and intron positions (Baxter *et al.,* 2003). In *Arabidopsis,* the two first clusters (HMA1, HMA2-4, respectively) are possible Zn/Cd/Co/Pb ATPases; the four others have a potential role in Cu/Ag transport. The first two characterised enzymes are members of this last subgroup. AtHMA7 (RAN1) has been identified as a copper transporter to ethylene receptors (Hirayama *et al.,* 1999; Woeste and Kieber, 2000). Recently AtHMA6 (PAA1) has been characterised as a Cu transporter, participating in the translocation of this metal to chloroplastic copper proteins (Shikanai *et al.,* 2003). Also, all eukaryotic HMAs characterised so far belong to this Cu/Ag subgroup.

Despite many members of the Zn/Cd/Co/Pb subgroup being known (Rensing *et al.,* 1998; Hou and Mitra, 2003; Lebrun *et al.,* 1994a and 1994b; Tsai *et al.,* 2002), the first characterization of one homologue in plants has only just been published (Mills *et al.,* 2003). These Authors showed that AtHMA4 induced an increased tolerance to Cd in wild-type *Saccharomyces cerevisiae* and restored Zn resistance of the *Escherichia coli zntA* mutant, which is highly sensitive to this metal. As predicted, AtHMA4 was unable to restore copper tolerance in the *copA* mutant context.

Therefore, it has been proposed to use the properties of these transporters in view to essentially increase resistance of plants to heavy metals:
- European Patent Application 1 136 558 proposes plants including sequences encoding one or more heterologous heavy metal transport and/or sequestration proteins of various prokaryotic or eukaryotic origins. The metal transport proteins are, for instance, membrane proteins such as P-type ATPases and preferably a bacterial P-type ATPase, such as cadmium ATPase. It is specified that such plants have improved heavy metal tolerance and possibly heavy metal accumulation; however, as regards the use of cadmium ATPase modified plant, the increased resistance of the plant to cadmium is the only characteristic pointed out.
- The PCT International Application WO 02/081707 discloses plant transformants with enhanced heavy metal resistance and decreased uptake of heavy metals. The P-type ATPase used to transform the plants is the ZntA, that is a P-type ATPase of *E. coli,* which pumps Pb(II)/Cd(II)/Zn(II) across the plasma membrane. More specifically it is considered, in said PCT International Application that biologically active ZntA-like heavy metal pumping ATPases include zinc-transporting ATPase (NC_000913), zinc-transporting ATPase (NC_002655), heavy metal-transporting ATPase (NC_003198), P-type ATPase family (NC_003197), cation transporting P-type ATPase from *Mycobacterium leprae* (GenBank #Z46257) and many others may be used. ZntA-transgenic plants and for instance ZntA-transgenic *Arabidopsis* plants are described; said plants showed enhanced resistance to lead and cadmium and the content of lead and cadmium was lower than in a wild-type plant. Therefore such transformed plants can grow in an environment contaminated with heavy metals; thus, said technique can be useful for generating crop plants with decreased uptake of harmful heavy metals. This is confirmed by the article in the name of LEE J. et al. (Plant Physiol., 2003), Inventors of said PCT International Application, which confirm that a bacterial heavy metal pump, ZntA, can be expressed in *Arabidopsis,* resulting in increased resistance to Pb(II) and Cd(II) and reduced heavy metal contents in the shoots of the transgenic plants. Therefore, both documents show that it is feasible to develop plants, *i.e*., transgenic ZntA *Arabidopsis* plants, with enhanced resistance and reduced uptake of heavy metals. They conclude that if ZntA can be introduced into crop plants, the resulting plants would be safer because they would contain less heavy metal in their shoots.

Therefore, the aim of the transgenic plants described in these two documents is not phytoremediation; thus the transgenic plants proposed in said documents cannot be used for phytoremediation and more specifically for phytoextraction, as defined here above.

- Mills R.F et al, (Plant J., 2003, precited) showed that AtHMA4, a P_{1B}-type ATPase of the Zn²⁺/Co²⁺/Cd²⁺/Pb²⁺ subclass induced an increased tolerance to Cd in wild-type *Saccharomyces cerevisiae* and restored Zn resistance of the *Escherichia coli* zntA mutant, which is highly sensitive to this metal. Even though they consider that there is considerable interest in metal transport in plants because of the implications for phytoremediation, it is only shown, in said document, that AtHMA4 is mainly expressed in roots of *Arabidopsis* and that it could function in planta as a detoxification system in epidermal root cells, existing at the plasma membrane to pump excess metals such as Zn or Cd into the rhizosphere. Therefore, they propose to use plants containing AtHMA4 for rhizofiltration.

It emerges from the foregoing that none of the disclosed methods proposes an efficient phytoremediation system for removal of heavy metals from soil, *i.e.,* a method using the phytoextraction process, which is particularly adapted to heavy metal removal from soil. Indeed, the prior disclosed methods have focused on the selection of plants that have a lower uptake of heavy metals than the wild-type, and that maintain healthy growth even in an environment contaminated with heavy metals and not on phytoremediation by phytoextraction.

Moreover, despite ever increasing interest and research in the field, several problems associated with phytoremediation remain. For instance, many metal-accumulating plants are unable to translocate a substantial proportion of the metal they acquire from their roots to other tissues.

Therefore, there is a need for plants able to extract heavy metals in harvestable parts thereof, in view to propose a cost-effective phytoremediation of heavy metals by phytoextraction.

The Inventors have found that, unexpectedly, plants overexpressing at least one P_{1B}-ATPase of the subfamily or subclass of the Zn²⁺/Co²⁺/Cd²⁺/Pb²⁺ transporters are good candidates for their use in a method of phytoremediation using the phytoextraction process, knowing that such genetically modified plants translocate efficiently the heavy metals in the shoots.

Therefore, in one aspect of the invention, genetically modified plants are provided which are able to accumulate heavy metals and translocate them to the shoots and more specifically to the stems and the leaves.

The preferred embodiments of this aspect of the invention include genetically modified plants, characterized in that they overexpress one or more than one copy of at least a sequence encoding a higher plant-origin P_{1B}-type ATPase of the Zn²⁺/Co²⁺/Cd²⁺/Pb²⁺ subclass. No need to say that "overexpress one or more than one copy of at least a sequence encoding a P_{1B}-type ATPase" means that said genetically modified plants overexpress at least one *additional* copy of the sequence encoding said ATPase. In a preferred embodiment, the plants are homozygous, and therefore contain an even number of copies of the added sequence (at least two). As described in the examples below, the homozygous plants can be obtained by self-crossing of transformed plants.

In a preferred embodiment, said P_{1B}-type ATPase is selected amongst HMA1, HMA2, HMA3 and HMA4. When the wild-type plant to be genetically modified for phytoremediation possesses HMA gene(s), additional copies of at least one of said endogenous gene(s) is (are) preferably introduced into said plant genome, in order of overexpress said endogenous gene(s). Alternatively, for example in case the wild-type plant is defective for the P_{1B}-type ATPase, the expression of which is desired in said plant for achieving phytoremediation of heavy metals, said P_{1B}-type ATPase can be selected from the group consisting of heavy metal ATPase HMA1 to HMA4 of *Arabidopsis thaliana* (*i.e.,* AtHMA1, AtHMA2, AtHMA3 and AtHMA4), whatever the ecotype, or from another plant species. The corresponding sequences of *Arabidopsis thaliana* are available on the following websites: http://mips.gsf.de or http://biobase.dk/~axe/Patbase.html.

The nucleic sequences which may advantageously be used are the ones of *Arabidopsis thaliana:*
- ecotype Ws (Wassilewskija): AY054390 (AtHMA2), AY434729 or AY055217 (AtHMA3), AF412407 (AtHMA4));
- ecotype Columbia: NM_119890 (AtHMA1), NM_119157 or AY434728 (AtHMA2), NM_119158 or AY434730 (AtHMA3), NM_127468 or AJ297264 (AtHMA4);
- ecotype Landsberg: AJ400906 (AtHMA1).

However, the invention includes also the use of corresponding sequences of other wild ecotypes of *Arabidopsis thaliana.* The skilled artisan may easily retrieve said equivalent sequences to the ones specified hereabove (AtHMA1-4) by appropriate alignment with said sequences.

According to another embodiment of the invention, said genetically modified plant overexpress one or more than one copy of at least two different sequences encoding two different higher plant-origin P_{1B}-type ATPases of the Zn²⁺/Co²⁺/Cd²⁺/Pb²⁺ subclass. According to this embodiment, the genetically modified plant preferably overexpresses at least both HMA3 and HMA4.

According to yet another embodiment of the invention, said genetically modified plant overexpress one or more than one copy of a sequence encoding a higher plant-origin P_{1B}-type ATPase of the Zn²⁺/Co²⁺/Cd²⁺/Pb²⁺ subclass and at least another sequence selected among sequences encoding (1) an enzyme involved in metal chelation (for example, phytochelatin synthase, glutathion synthetase or gamma-glutamylcystein synthase) or (2) another metal transporter such as YCF1 or other ABC transporters.

Said AtHMA sequence can be inserted in an appropriate vector, said AtHMA sequence being operably linked to and under the regulatory control of a plant-expressible transcription and translation regulatory sequence, such as a plant specific promoter and more preferably the CaMV35S promoter, and said vector is introduced in the selected plants with known methods. Said vector is advantageously an *Agrobacterium*/plant shuttle vector.

Preferably said vector may be introduced in plants, such vector allowing the transformation of plants with the *Agrobacterium tumefaciens* technology. Transformed plants can then be self-crossed for obtaining homozygous stable lines.

According to the invention, the plants to be used are selected in the group consisting of *Brassica juncea, Poplar, Nicotiana tabacum.*

The instant invention also relates to a recombinant vector able to transform plants, characterized in that said vector includes one or more than one copy of at least a sequence encoding a higher plant-origin P_{1B}-type ATPase of the Zn²⁺/Co²⁺/Cd²⁺/Pb²⁺ subclass (heavy metal pumping ATPases) in view that said transformed plants overexpress at least one of said P_{1B}-type ATPase of the Zn²⁺/Co²⁺/Cd²⁺/Pb²⁺ subclass. The ATPase encoded by the sequence introduced in a vector according to the invention is chosen so that it is able to accumulate heavy metals and translocate them to the shoots of a plant when expressed in said plant. Hence, ZntA is not an appropriate ATPase for use according to the invention. Examples of ATPases that can be advantageously used are eukaryotic P_{1B}-ATPases HMA1, HMA2, HMA3 and HMA4, especially those originating from plants close to the plants that are used for phytoremediation.

Advantageously, said coding sequences are operably linked to and under the regulatory control of a plant-expressible transcription and translation regulatory sequence, such as a plant specific promoter.

In a particular embodiment, the recombinant vector according to the invention comprises a first sequence encoding HMA3, and a second sequence encoding HMA4. As described in Example 3 below, these two ATPases act synergistically to accumulate heavy metals - especially Co, Pb and Cd - in the shoot. The invention thus also relates to a set of recombinant vectors as described above, comprising at least a first vector encoding HMA3, and a second vector encoding HMA4.

The invention also relates to genetically transformed plant cells or plants overexpressing at least one higher plant-origin P_{1B}-type ATPase of the Zn²⁺/Co²⁺/Cd²⁺/Pb²⁺ subclass, obtainable by transforming plant cells or plants with said vector or set of vectors.

The instant invention also relates to a method of producing genetically modified plants which overexpress at least a P_{1B}-type ATPase of the Zn²⁺/Co²⁺/Cd²⁺/Pb²⁺ subclass, comprising:
- preparing at least a recombinant vector as defined here above, *i.e*., comprising one or more than one copy of at least a sequence encoding a higher plant-origin P_{1B}-type ATPase of the Zn²⁺/Co²⁺/Cd²⁺/Pb²⁺ subclass, operably linked to and under the regulatory control of a plant-expressible transcription and translation regulatory sequence and
- introducing said recombinant vector into a plant cell or plant tissue to produce a genetically modified plant cell or a genetically modified plant tissue.

An additional step of self-crossing may be performed to obtain homozygous lines.

In another aspect of the invention, the genetically modified plants as described above are used for extracting heavy metals from the environment (*i.e*., soil, water, *etc.,* in particular a contaminated soil). Advantageously, plants overexpressing both HMA3 and HMA4 are used for phytoextraction of Co, Cd or Pb from an environment contaminated with said metal(s). Therefore, the instant invention also relates to a method of phytoremediation of heavy metals from soil, characterized in that it includes:
- a step of planting genetically modified plants as described here above, in an area containing soil contaminated with at least one heavy metal and
- collecting and removing plant tissues from said genetically modified plants at appropriate time intervals.

The preferred embodiments of this aspect of the invention involve the extraction of at least one of the following heavy metals: Zn, Co, Cd or Pb, from soil. When plants overexpressing both HMA3 and HMA4 are used, it is noted that the synergistic effect of this co-expression is achieved for Co, Cd and Pb, but that the remediation capacity of said plant concerning Zn is not significantly improved by the overexpression of HMA3.

The entire plant might be removed after it has been allowed to grow on metal-containing environment (*i.e*., soil, water, *etc*.), thereby incorporating those metals into its tissues. However, in many of the preferred embodiments, it may be taken advantage of the ability of said genetically modified plants as described here above to sequester substantial proportions of its accumulated metal in stems and leaf tissues.

Thus, said genetically modified plants are planted in contaminated soil and allowed to grow and scavenge the cited metals from the soil.

Then, at appropriate time intervals, the metal containing tissues and more preferably leaves and possibly branches are removed from the plant, allowing the remaining plant tissues to survive.

The collected plant tissues are removed from the growing area and properly disposed, so that the metal containing tissues are not allowed to reassimilate in the soil.

In another embodiment of the instant invention, said heavy metals may be extracted in the Mⁿ⁺ state from said plant tissues by known methods.

In yet another embodiment of the instant invention, said heavy metals are extracted from ashes obtained after having burnt the collected metal containing tissues, said metal being in the M⁰ state.

The burning operation is made under controlled environmental process.

The heavy metals, obtained in a M⁰ state, may be, advantageously, directly recycled.

The Table below establishes the correspondences between the sequence numbers as they appear in the sequence listing and the name of the various sequences.

| Designation | SEQ ID |
|---|---|
| Rev6HMA4 | SEQ ID NO:1 |
| Rev5HMA4 | SEQ ID NO:2 |
| Rev4HMA4 | SEQ ID NO:3 |
| Rev3HMA4 | SEQ ID NO:4 |
| Rev2HMA4 | SEQ ID NO:5 |
| Rev1HMA4 | SEQ ID NO:6 |
| 1HMA4 | SEQ ID NO:7 |
| Rev14HMA4 | SEQ ID NO:8 |
| TopoFV1for | SEQ ID NO:9 |
| TopoFV1rev | SEQ ID NO:22 |
| HMA4For230 | SEQ ID NO:10 |
| Rev2bisHMA4 | SEQ ID NO:11 |
| Actin8F | SEQ ID NO:12 |
| Actin8R | SEQ ID NO:13 |
| Rev18HMA4 | SEQ ID NO:14 |
| 10HMA4 | SEQ ID NO:15 |
| Rev8HMA4 | SEQ ID NO:16 |
| 22HMA4 | SEQ ID NO:17 |
| ΔHisStopHMA4 | SEQ ID NO:18 |
| HMA4D401Afor | SEQ ID NO:19 |
| HMA4D401Arev | SEQ ID NO:20 |
| 9HMA4 | SEQ ID NO:21 |

In addition to the preceding arrangements, the invention also comprises other arrangements which will emerge from the description which follows, which refers to exemplary embodiments of the method which is the subject matter of the present invention as well as to the appended drawings, in which:

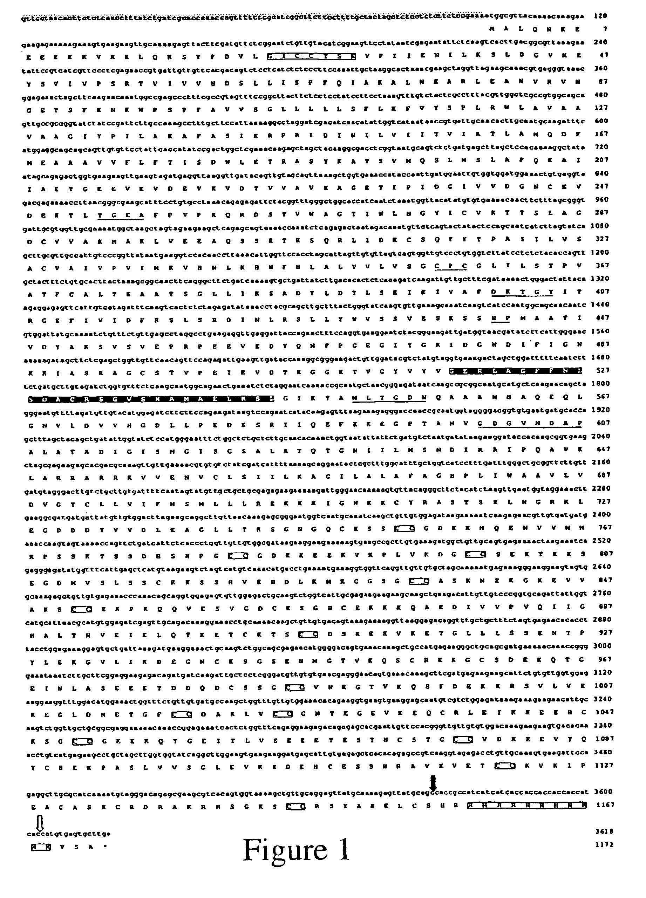

- **Figure 1****:** Nucleic and amino acid sequences of AtHMA4 from the Ws ecotype (accession number AF412407). The grey highlighted characters correspond to the 5' UTR. The N-terminal heavy metal motif, the numerous cysteine doublets and the His stretch are boxed; the highly conserved sites are underlined. The amino acid sequence deleted in the alternative spliced form is black highlighted. The position of the stop insertion for the *Athma4*Δ*His* form and its GFP fusion is indicated by the solid black vertical arrow while the empty one corresponds to the GFP fusion position of *AtHMA4* and *Athma4as.*

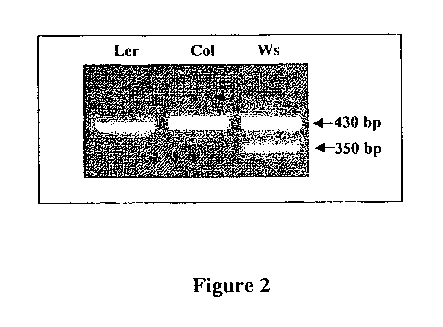

- **Figure 2****:** Evidence for the existence of an alternative spliced form of the cDNA of *AtHMA4* in the Ws ecotype, absent in Ler and Col-0 ecotypes. RT were carried out with 4 µg total leaf RNA.

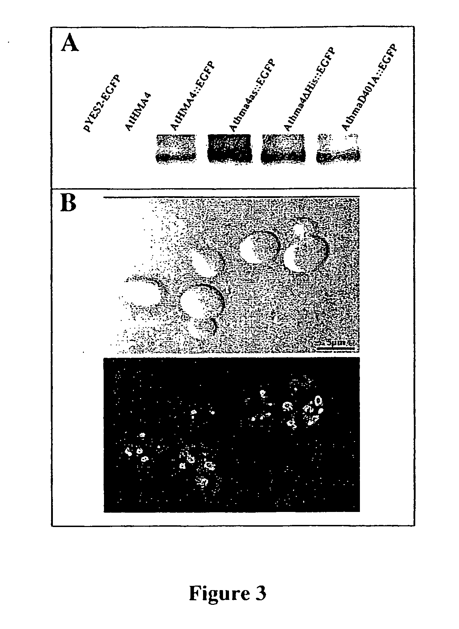

- **Figure 3****: A**: Western blot analysis of the microsomal fractions of the different transformant strains expressing the chimeric constructions and AtHMA4 without fusioned EGFP. 50 µg protein was loaded on 8 % SDS-PAGE for each fraction. The resolved proteins were electrotransferred on nitrocellulose membrane and revealed with anti-EGFP horseradish peroxidase antibodies. **B**: Confocal microscopy observations of yeast cells expressing AtHMA4::EGFP. Top, transmission image; bottom, EGFP fluorescence image.

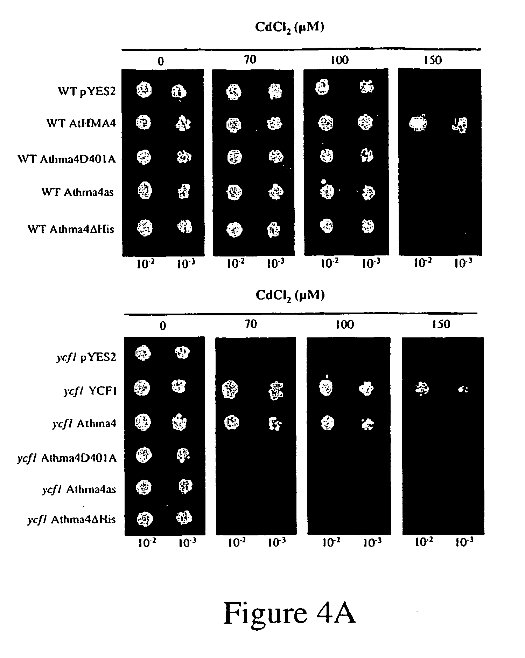

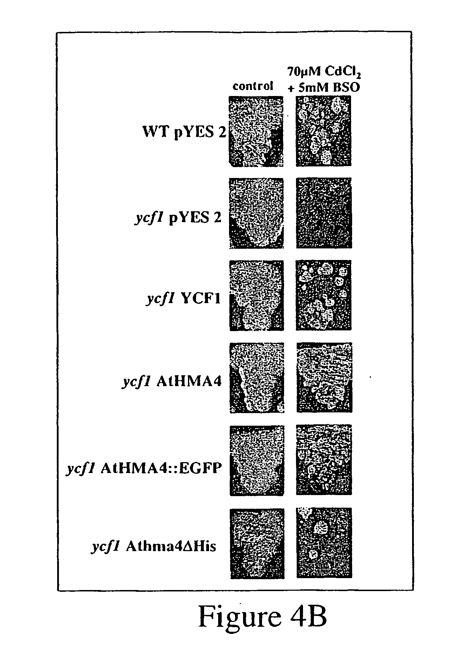

- **Figure 4****: A**: AtHMA4, but not Athma4D401A, Athma4as and Athma4ΔHis, confers an increased tolerance to Cd and is able to functionally substitute the *ycf1* mutant strain of *S. cerevisiae.* The various strains were grown on solid SC minimal medium, supplemented with CdCl₂ at the indicated concentrations. Plates were incubated at 30 °C for 4 days. Upper part (wild-type): pYES2 only-, AtHMA4-, Athma4D401A-, Athma4as- and Athma4ΔHis-transformants of the wild-type strain BY4741. Bottom part: same as upper part, except that the constructs are expressed in the *ycf1* mutant strain (*ycf1* YCF1 indicated a complementation of the mutant strain *ycf1* by pYES2-*YCF1*). **B**: Metal tolerance conferred by AtHMA4 does not implicate a gluthatione S-conjugated form of Cd. The different strains were grown on solid SC minimal medium without (control) or supplemented with 70 µM CdCl₂ and 5 mM BSO during 4 days at 30 °C.

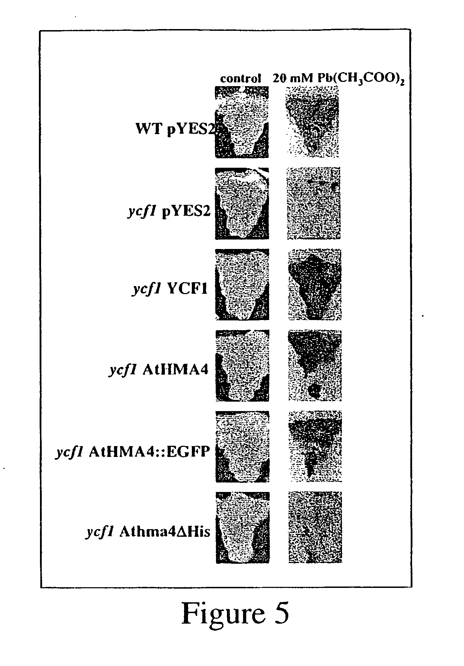

- **Figure 5****:** Expression of AtMHA4 or AtHMA4::EGFP, but not Athma4ΔHis, induces a functional substitution of ycf1 in the presence of Pb. The strains were grown on solid SC minimal medium without (control or supplemented with 20 mM Pb(CH₃COO)₂ (conditions and strains were the same as in Fig. 4).

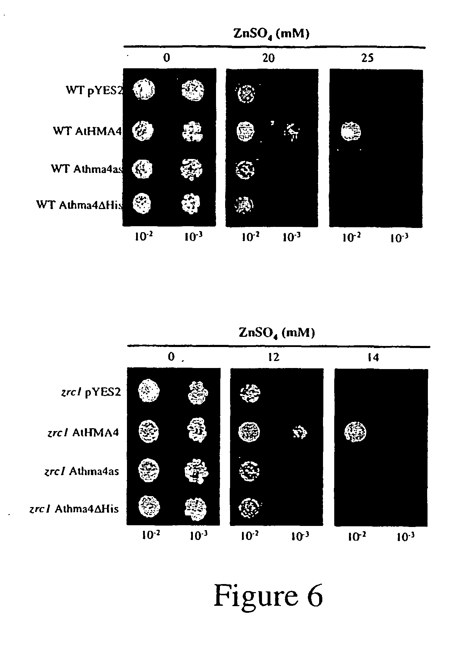

- **Figure 6****:** AtHMA4 confers a slightly enhanced tolerance to Zn and was able to partially substitute the *zrc1* strain. Strains and culture conditions were the same as in Fig. 4. ZnSO₄, 7H₂O was added to the medium at the indicated concentrations.

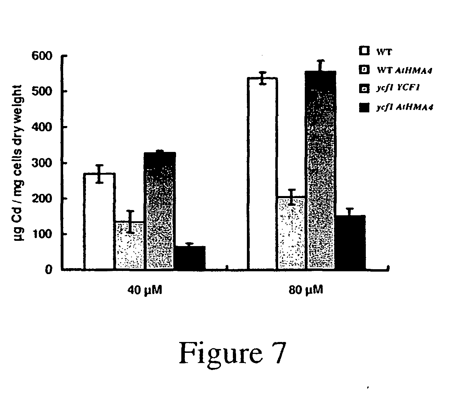

- **Figure 7****:** Cd content of a 48 h culture of pYES2-only (WT) or AtHMA4- (WT *AtHMA4*) transformant of the wild-type; YCF1- (*ycf1 YCF1*) or AtHMA4- (*ycf1 AtHMA4*) transformant of the mutant *ycf1* strain. Error bars represent the SE from five independent experiments.

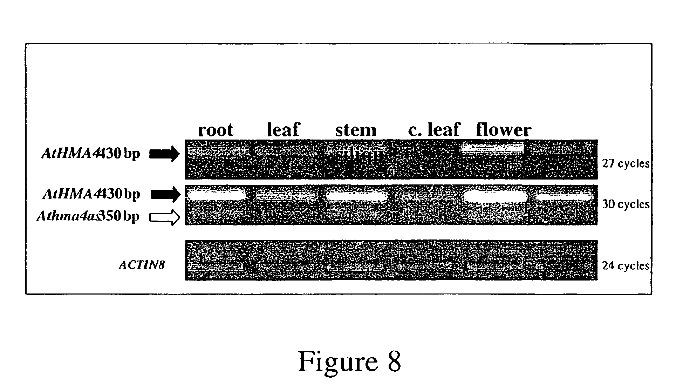

- **Figure 8****:** Expression profile of *AtHMA4* (and *Athma4as*) determined by RT-PCR on RNA extracted from various tissues. The results of RT-PCR are presented after 27 and 30 cycles to better visualise the expression of the alternative spliced form (*Athma4as,* fragment of 350bp size).

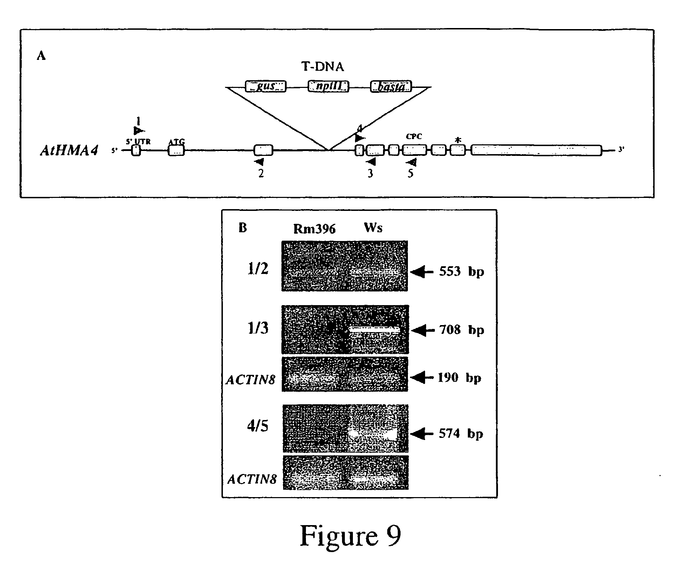

- **Figure 9****: A:** Exon/intron map of *AtHMA4* including the 5'UTR and position of the T-DNA insertion of the Rm396 mutant. The asterisk on the ninth exon (including the 5'UTR) indicates the position of the deletion in the alternative spliced form of the cDNA (*Athma4as*). The arrows indicate the different primers used to control the existence of a transcript by RT-PCR, using RNA extracts from Rm396 and Ws. **B:** The T-DNA insertional Rm396 line is a true K.-O. mutant for *AtHMA4.* The RT-PCR was carried out on RNA with different primer pairs, specific to *AtHMA4.* Upper: oligonucleotides 1 (1HMA4: SEQ ID NO:7) and 2 (Rev6HMA4: SEQ ID NO:1); middle: I and 3 (Rev18HMA4: SEQ ID NO:14); lower: 4 (10HMA4: SEQ ID NO:15) and 5 (Rev8HMA4: SEQ ID NO:16).

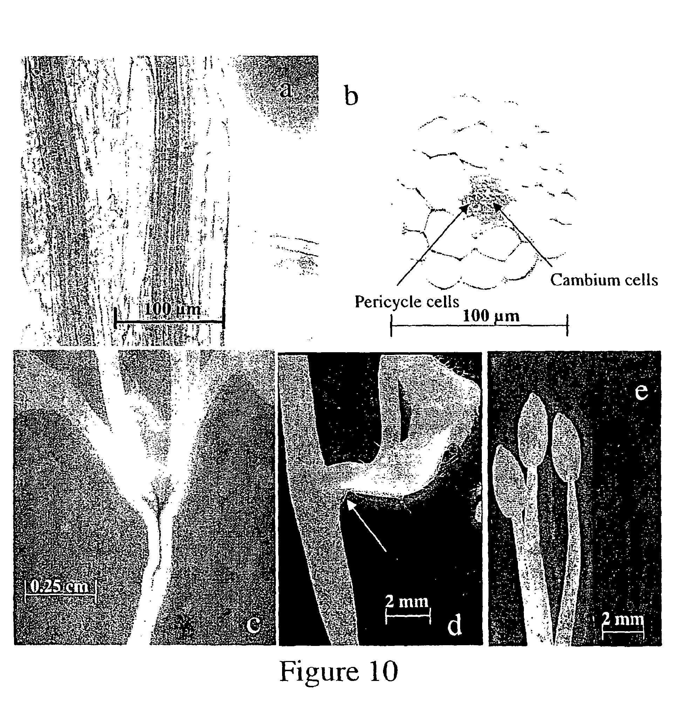

- **Figure 10****:** Expression profile of GUS activity *in plants.* a: roots; b: root slice; c: plantlet; d: stem and cauline leaf; e: stamens.

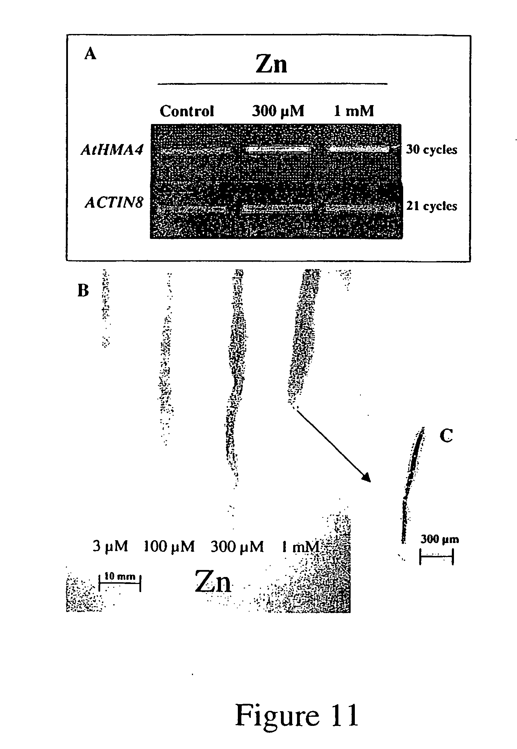

- **Figure 11****:** *AtHMA4* expression is up-regulated by Zn treatment. **A:** RT-PCR amplification on total RNA. **B:** GUS staining on root system. The inset corresponds to a binocular observation of a 1 mM Zn-treated root. Plants were grown hydroponically and treated for 24 h with ZnSO₄ at the indicated concentrations.

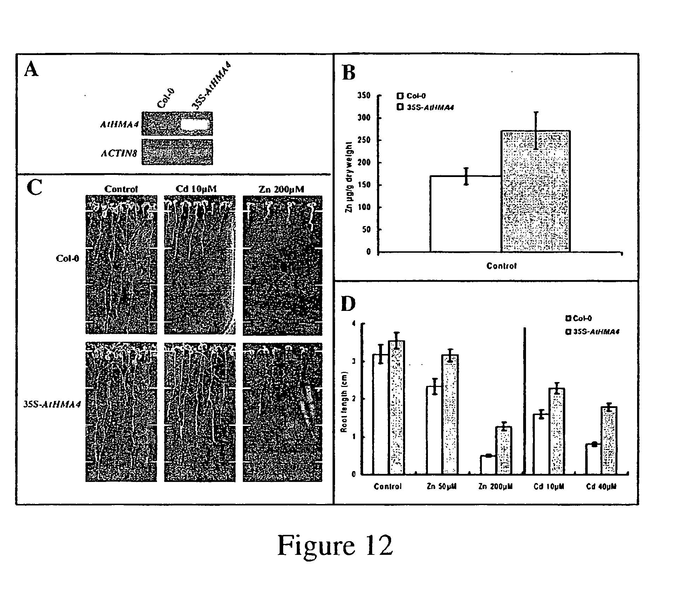

- **Figure 12****:** The overexpression of AtHMA4 confers an increased tolerance to metals. **A:** Overexpression of *AtHMA4* determined by RT-PCR carried out with RNA extracted from leaves. **B:** Zn content of leaves from wild-type (Col-0) or overexpressing (*35S-AtHMA4*) plants hydroponically grown during 8 days on standard nutrient solution (Zn 3 µM), determined by ICP measurements. **C:** Root length of vertically grown plantlets. The photography of plates are realised 15 days after germination. **D:** The graph represents the results of one representative experiment of 3 with one of the two lines analyzed in detail. Values are mean of measurements of 75-100 roots, and error bars represent SEM (P < 0.005).

It should be clearly understood, however, that these examples are given solely by way of illustration of the subject of the invention and do not in any manner constitute a limitation thereto.

### EXAMPLE 1: MATERIALS AND METHODS

### - Plant material, culture media, and extraction and measurement of total RNA.

Plants were grown in a controlled-environment (8 h photoperiod of 300 µmol.m⁻².s⁻¹, 22 °C and 70 % relative humidity), in Murashige and Skoog medium which consisted of a ½ strength (MS/2) nutrient solution (Murashige and Skoog, 1962), plus 1 % (w/v) sucrose, and 0.7 % (w/v) Bacto agar to produce a solid medium. Germination of surface-sterilized seeds of *Arabidopsis thaliana,* ecotypes Wassiliewskija (Ws), Columbia (Col-0) and Landsberg *erecta* (Ler) occurred on the solid medium. After 2 weeks, the young plants were placed on sand, saturated with the same nutrient solution, for an additional 3 weeks period and finally transferred to a hydroponic culture system. When plants were treated with different metals (*in vitro* or hydroponically), they were supplied at various concentrations as indicated in the figure legends, in addition to that in the nutrient solution.

Total RNA was extracted from various tissues (root, leaf, stem, cauline leaf, flower and silique) according to Verwoerd *et al.* (1989), and the RNA concentration determined using UV absorption at 260 nm (BioPhotometer, Eppendorf, Hamburg, Germany).

### - Cloning of the cDNA of AtHMA4

Two successive 5' RACE-PCR were performed (5' RACE System, GibcoBRL^{®}, Carlsbad, CA) according to the manufacturer's instructions. In the first cycle of RACE, the cDNA was amplified with the gene specific oligonucleotide primer Rev6HMA4 (5'-GACCAATATGTTGATGTCGATCC-3') (SEQ ID NO:1) situated in the 2^{nd} predicted exon. The two successive semi-nested PCR were carried out using the Expand High-Fidelity System (Roche, Mannheim, Germany) with the primers Rev5HMA4 (GGCTTTGGCAAGAATCGGATAG-3') (SEQ ID NO:2) and Rev4HMA4 (5'-GCGGCAACTGCTGCCACGGCGAGCC-3') (SEQ ID NO:3), respectively. Reaction conditions were 3 min at 94 °C, and then 35 cycles for 30 sec at 94 °C, 30 sec at 53 °C or 67°C, 1 min at 68 °C. The last cycle was followed by an extended elongation step of 5 min at 68 °C. The amplification fragments were cloned in pGEM-T^{®} Easy vector (Promega, Madison, WI). The second round of 5' RACE was performed as described above. The different primers used were Rev3HMA4 (5'-CTTCTTCACTTTCTTTTTCTCTTCTTC -3) (SEQ ID NO:4) for the cDNA amplification, Rev2HMA4 (5'-GTAGCAAAAGGAAGAAGCCGATG-3') (SEQ ID NO:5) and Rev1HMA4 (5'-CTGGTTTGGTGCGATCAGATAAAGG-3') (SEQ ID NO:6) were used in the two successive semi-nested PCR, with annealing temperature of 55 °C and 58°C, respectively.

First-strand full-size cDNA synthesis was performed with the ThermoScript™ RT-PCR system (Invitrogen™, Carlsbad, CA) according to the manufacturer's instructions using 4 µg of total leaf RNA and an oligo (dT)₂₀ primer. PCR was then carried out with Platinum^{®} *Pfx* (Invitrogen™) with primers corresponding to the 5'-UTR and 3' end of the predicted coding sequence present in the BAC clone (5'-end primer 1HMA4 (5'-CACTTCTCTCAACCTTTATCTGAT-3') (SEQ ID NO:7) and 3'-end primer Rev14HMA4 (5'-GTTATTCAATCAATCTCCATCAAG-3') (SEQ ID NO:8)). The PCR reaction conditions were as described above, except the hybridization temperature (50 °C) and the final elongation step (10 min). The amplification fragment of 3.6 kb was directly cloned in the pCR^{®}-XL-TOPO vector (Invitrogen™). The same procedure was used for the cloning of the cDNA of the alternative spliced form (*AtHMA4as*).

### - Obtaining of AtHMA4 overexpressing lines

After digestion by *Eco*RV, the CaMV35S cassette was subcloned in the *Eco*RV / *Stu*I sites of the pGreen0179 binary vector. A *Sma*I site was added at the 3'-end of AtHMA4 by cloning the reverse-complement primer pairs TopoFV1for (SEQ ID NO:9)/rev (SEQ ID NO:22) in the *Not*I site of the pCR^{®} XL-TOPO vector. The cDNA of AtHMA4 was then extracted by *Bam*HI / *Sma*I digestion and cloned at the same sites in the pGreen-CaMV35S vector. This construction was introduced by electroporation in AGL1 cells of *A. tumefaciens.* The agrotransformation of plants was carried out by the floral dip method (Clough and Bent, 1998). The transformant plants were selected on solid medium supplemented with hygromycin B 30 µg / ml.

### - DNA fragment resolution

The DNA fragments were electrophoresed on 1 % (w/v) agarose gels prepared in TAE buffer in the presence of ethidium bromide (Sambrook *et al.,* 1989). The DNA was visualised under UV light using a GeneGenius GG-X apparatus (Syngene, Cambridge, UK). Extraction and purification of DNA fragments were obtained with the NucleoSpin^{®} Extract kit (Macherey-Nagel, Düren, Germany).

### - Sequencing

The "BigDyes" method (PE Biosystems, Foster City, CA) was used with a Perkin Elmer ABI Prism 310 sequencer.

### - Expression analysis of AtHMA4

* *RT-PCR.* Total RNA extractions and first-strand cDNA synthesis were carried out as described above. The same amount of RNA (4 µg) from all the organs analysed was used for RT-PCR with an oligo(dT)₂₀ primer. Primers HMA4For230 (5'-CGCAGCTTGCTTTACTGGGTATCAAGTGTTGAAAG-3') (SEQ ID NO:10) and Rev2bisHMA4 (5'-CATCTAAAACATTCCCTAGCTGTTCTTGAGC-3') (SEQ ID NO:11) were used for PCR performed in the same conditions as described above, except the hybridization temperature (65 °C) and the number of cycles (26 cycles), which was chosen to be in the mid-log phase of amplification yield. As a control, PCR of *actin8* gene was carried out on the same cDNA samples using the primers Actin8F (5'-GTGGTCGTACAACCGGTATTGTGTTGGACTCTGGTG-3') (SEQ ID NO:12) and Actin8R (5'-ACGCTGTAACCGGAAAGTTTCTCACATAGTGCACAAATGAC-3') (SEQ ID NO:13). These primers were added to the PCR reaction tubes 5 cycles later. The experiment was repeated 3 times on independently isolated material. Three RT-PCR were carried out, to control the Rm396 mutant line, with primer pairs located upstream, on both sides and downstream relative to the T-DNA insertion position. The RT was carried out with the gene specific primer Rev6HMA4 (SEQ ID NO:1) for the first one; the first strand cDNA was used in PCR reaction with the primers 1HMA4 (SEQ ID NO:7) and Rev6HMA4 (SEQ ID NO:1). The PCR reactions for the other two were performed with oligo(dT)₂₀ cDNA using primer pairs 1HMA4 (SEQ ID NO:7)/ Rev18HMA4 (5'-GCCTTTTGTGGAGCTAAGCTC-3') (SEQ ID NO:14) and 10HMA4/ Rev8HMA4 (5'-ATGGAGGCAGCAGCAGTTGTGTTCC-3' (SEQ ID NO:15)/ 5'-GGGACAACCACTGACTAACACAAC-3' (SEQ ID NO:16)), respectively. PCR of the *actin8* gene was carried out on the same cDNA samples as described above.

* *GUS activity.* Plants or organs at different stage of their development were examined for GUS activity according to the procedure described by Jefferson *et al.,* 1987. The samples were immersed in the GUS staining solution (50 mM NaPO₄, pH 7.0, 0.01 % (w/v) Triton-X100, 1 mM K₃Fe(CN)₆, 1 mM K₄Fe(CN)₆ and 1 mg.ml⁻¹ 5-bromo-4-chloro-3-indolyl-β-D-glucuronide (X-Gluc)), vacuum-infiltrated and incubated at 37 °C for 6 hours. Pigments were then washed out by incubation of stained sample in 2-3 changes of 70 % (v/v) ethanol at 60 °C for 2 h. Plantlet roots were desiccated by successive ethanol baths of rising concentrations, for 30 min each and then fixed in resin, before transverse sections were cut.

### - Yeast strains, growth conditions and transformation

The *S. cerevisiae* reference strain BY4741 (*MATa; his3*Δ*1; leu2*Δ*0; meta15*Δ*0*; *ura3*Δ*0*), and mutant strains Δ*ycf1* (like BY4741 except *YDR135c: : kanMX4), Δzcr1* (like BY4741 except *YMR243c::kanMX4*) and Δ*cot1* (strain BY4742: *MATa; his3*Δ*1; leu2*Δ*0; lys2*Δ*0; ura3*Δ*0; YOR316c::kanMX4)* were provided from Euroscarf (Frankfurt, Germany). All yeast strains were grown in YPD medium (1 % (w/v) yeast extract, 2 % (w/v) peptone, 2 % (w/v) dextrose). Synthetic complete (SC) minimal medium lacking uracil was used for the selection and maintenance of yeast strains transformed with plasmid (0.67 % (w/v) yeast nitrogen base, 0.19 % (w/v) drop-out without uracil, 2 % (w/v) dextrose) (all products from Sigma, St Louis, MO).

*AtHMA4* and the alternative spliced form (*Athma4as*) were cloned into the *Not*I site of the yeast expression vector pYES2 under a galactose-inducible promoter. A truncated version of AtHMA4 lacking the C-terminal His₁₁ stretch was constructed. A PCR, using the *PfuUltra*™ (Stratagene^{®}, Cedar Creek, TX), was carried out with the primers:
- 22HMA4: (5'-GAGAGTGTTGGAGACTGCAAGTCTGGTCATTGCCAGAAG-3') (SEQ ID NO: 17) and
- ΔHisStopHMA4: (SEQ ID NO:18)
   (5'-AAGGAAAAAAGCGGCCGCAAAAGGAAAATTAGCTGCATAACTCTTTTGCATA AC-3'). This 860 bp fragment, digested by *Bsg*I and *Not*I*,* was cloned in place of the same fragment in the full-size cDNA. This cDNA was then digested by *Eag*I and cloned in pYES2 into the *Not*I site. These 3 cDNA were also cloned in the same vector in frame with the *EGFP* gene in 3'. For these clonings, the *AtHMA4* and *Athma4as* cDNA were first digested by *Dra*III at the 3' end of the genes, the end being blunted by S1 nuclease treatment. Then the fragments were excised from the pCR^{®}-XL-TOPO vector by *Eco*RI digestion and finally cloned into the *Eco*RI and *Sma*I sites of the pYES2-GFP vector, in frame with the *EGFP* gene. Also, to create the *Athma4*Δ*His::EGFP* fusion, a PCR amplification was carried out on the cDNA of *AtHMA4* with the primers 22HMA4 (SEQ ID NO:17) and ΔHisHMA4 (5'-AAGGAAAAAAGCGGCCGCAAAAGGAAAACCCGGGGCTGCATAACTCTTTTGCAT AAC-3') (SEQ ID NO:18) which delete the His coding fragment and create a *Sma*I site. The amplification fragment was cloned in place of the corresponding fragment in the native cDNA into the *Bsg*I and *Not*I sites. The cDNA of *Athma4*Δ*His* was then cloned into the *Eco*RI and *Sma*I sites of the pYES2-GFP vector. By sequencing, the fusions were controlled for the 3 constructions. The Δ*ycf1* mutant strain was complemented by a *YCF1::EGFP* fusion cloned into the *Sac*I and *Not*I sites of the pYES2 vector. A site-directed mutagenesis was carried out using the QuickChange^{®} II XL Site-directed Mutagenesis Kit (Stratagene^{®}) on the *AtHMA4::EGFP* construct in pYES2 as a template with the primers pairs:
- HMA4D401A for: (SEQ ID NO:19)
   (5'-GATCAAGATTGTTGCTTTCGCTAAAACTGGGACTATTACAAGAGG-3') and
- HMA4D401 Arev: (SEQ ID NO:20)
   (5'-CCTCTTGTAATAGTCCCAGTTTTAGCGAAAGCAACAATCTTGATC-3') to create a mutant where the aspartate-401 residues from the conserved domain DKTGT was replaced by an alanine, giving the mutant *Athma4D401A::GFP.*

The yeast strains were transformed with pYES2 alone or with *AtHMA4-, Athma4D401A-; Athma4as-, Athma4ΔHis-; AtHMA4::GFP-, Athma4D401A::GFP-, Athma4as::GFP-* or *Athma4ΔHis::GFP*-pYES2, according to the manufacturer instructions (Invitrogen™).

### - Assays for metal tolerance of yeast

Yeast cells were first pre-cultured overnight at 30 °C in SC minimal medium without uracil (2 % (w/v) raffinose instead of dextrose), then transferred to the induction medium at an O.D.₆₀₀ₙₘ = 0.4 for 24 h (in same minimal medium except that 1 % (w/v) raffinose and 2 % (w/v) galactose were added). For solid media, 2 % (w/v) agar was added. The different metal solutions were added to the liquid or solid media at the concentrations indicated in the figure legends. For the drop-tests on solid media, 2 µL of the yeast cultures at O.D.₆₀₀ₙₘ = 2.0 were spread (1.6 10⁴ cells) and three 10-fold serial dilutions were made. Plates were incubated at 30 °C for 4 days. For the liquid cultures, yeast cells were diluted to O.D.₆₀₀ₙₘ = 0.4 and grown at late log phase.

### - GFP fluorescence observations by confocal microscopy

The different transformant yeast strains were observed for GFP fluorescence using a confocal laser scanning microscope (CLSM, Fluoview Olympus, France) fitted with a krypton/argon laser. Fluorescence excitation was achieved with a dichroic filter (488 nm) and recording performed at 510 - 550 nm. Observations were carried out with an objective lens (UplanApo) at a magnification of x 100 / 1.35 na, under oil immersion.

### - Measurement of metal content of yeast cells

Yeast cells were grown in the same conditions as described for metal tolerance assays. After 48 h of growth in 15 mL of induction medium supplemented with heavy metals, the OD₆₀₀ₙₘ was determined. The cells were collected by centrifugation, then washed three times with 10 mM EDTA, pH 7.5, and finally with water. The washed pellet was dried overnight at 80 °C and then mixed with nitric acid for mineralization using a MARS X microwave apparatus (CEM, Matthews, NC). Finally, heavy metals contents were determined using an ICP-AES Vista MPX apparatus (Varian, San Diego, CA).

### - Preparation of soluble and microsomal protein fractions

An overnight pre-culture of transformed yeast was carried out in 3 mL of SC-URA minimal medium at 30 °C, 270 rpm. Cells were harvested by centrifugation (5 min, 6,000 rpm), diluted at 0.4 OD₆₀₀ₙₘ in 50 mL induction medium, and were grown for further 24 h. All further procedures were carried out at 4 °C. Yeast cells were harvested by centrifugation (10 min at 7,000 g), and suspended in 20 mL TEK buffer (50 mM Tris HCl pH 7.5, 1mM EDTA, 100 mM KCl). After centrifugation for 10 min at 7,000 g, the pellet was suspended in 2 mL TES buffer (50 mM Tris HCl pH 7.5, 1 mM EDTA, 0.6 M sorbitol, 1% (w/v) BSA, 2mM β-mercaptoethanol, 10 mM Na₂SO₄, and a "Complete" protease inhibitor cocktail (Roche Diagnostics)). Cells were then broken by vigorous mixing with acid-washed glass beads (425-600 µm, Sigma). The glass beads were washed four times with TES buffer, the washing fractions pooled and then centrifuged for 15 min at 8,000 rpm. Then, the supernatant was ultracentrifuged for 45 min at 100,000 g and the microsomal fraction was suspended in 500 µl TEG buffer (50 mM Tris HCl pH 7.5, 1mM EDTA, 30% (v/v) glycerol). Protein concentrations were measured with the Bradford method (Sigma).

### - SDS-PAGE and immunoblotting

Protein samples (50 µg) were rapidly thawed and mixed with denaturing buffer (1/4; v/v) containing 20 mM Tris HCl pH 8.0, 2% SDS (w/v), 10% β-mercaptoethanol (v/v), 20% glycerol (v/v), and bromophenol blue. Samples were heated at 60 °C for 30 min, cooled, and then loaded on a 8% SDS-PAGE. After electrotransfer of the proteins onto a nitrocellulose membrane (BioRad, Hercules, CA), the blot was incubated with a primary monoclonal antibody raised against the EGFP diluted to 1/1000 (v/v) (Clontech, Palo Alto, CA). Revelation was obtained with a secondary horseradish peroxidase antibody diluted to 1/5000 (v/v) (Sigma) and the SuperSignal^{®} West Pico Chemiluminescent Substrate (Pierce, Rockford, IL). All steps were performed according to the manufacturer's instructions.

### EXAMPLE 2: RESULTS

### - Cloning and sequence analysis of AtHMA4 from the Ws ecotype.

A study was conducted on the Wassiliewskija ecotype (Ws) since a T-DNA insertional mutant of *AtHMA4* was identified in the INRA Versailles collection. It was first determined whether the predicted ATG in the BAC clone AC002392 corresponded to the one in Ws. Two successive 5' RACE-PCR on total RNA extracted from plant tissues were performed (see Example 1). In the first one, the cDNA was obtained with a reverse primer designed in the second predicted exon. By two successive semi-nested PCR, two amplification products were resolved, with 516 bp and 488 bp sizes, respectively. By sequencing, it was verified that these fragments correspond to *AtHMA4.* The second round of 5'RACE, using primers corresponding to the new found sequence, did not enable another upstream transcription fragment to be obtained indicating that the 5' end of the mRNA of *AtHMA4* was determined. In addition to the coding sequence predicted from the genomic DNA of Columbia (Col-0) ecotype; a 99 bp 5'-UTR was found. The full-length cDNA (3618 bp) was then obtained by a two steps RT-PCR in a unique fragment, cloned in the pCR^{®}-XL-TOPO vector and sequenced (Genbank accession number AF412407) (Fig. 1). Some nucleic acid differences with respect to the sequence of the BAC clone from Col-0 were observed. The ecotypic differences between Ws and Col-0 were controlled by various independent PCR on genomic DNA and cDNA. Finally, four base changes were detected, resulting in amino acid differences (H *vs.* Q in position 537; K *vs*. R in 805; N *vs.* T in 970 and K *vs.* T in 1056).

Several RT-PCR experiments pointed to an alternative spliced form of the cDNA of *AtHMA4* (Fig. 2). With the same pair of primers, two amplification fragments were obtained with a size of about 450 bp and 350 bp, respectively. These two fragments were cloned and sequenced (442 and 358 bp), both corresponding to *AtHMA4.* To exclude any problem during the reverse transcription reaction, such as a stable hairpin loop of the mRNA, various conditions were tested (different reverse transcriptases and/or reverse primers more or less closely located downstream of the alternative splicing part of the gene). In all conditions tested, the spliced product was obtained in which a 84 bp fragment, between positions 1652 and 1735 of the nucleic sequence, was absent (Fig. 1, bases in square brackets and highlighted amino acids). The canonical limits of intron (5'-begin GT, 3'-end AG) were present at both sides of this fragment but different software for exon/intron prediction (GeneFinder, GeneScan) did not indicate this sequence as a potential intron. In the spliced form of the transcript the reading frame was conserved. Using RT-PCR, the full-size cDNA corresponding to this spliced form was amplified, cloned in the pCR^{®}-XL-TOPO vector, and called *Athma4as.* Such spliced form of the cDNA was never observed with RNA extracted from the Landsberg *erecta* or Columbia ecotypes (Fig. 2), although the nucleic acid sequences around the splicing sites were identical for Col-0 and Ws.

### AtHMA4 expression in yeast

*AtHMA4, Athma4D401A* and both truncated forms, *Athma4as* and *Athma4*Δ*His,* were subcloned in the pYES2 vector and expressed in the *Saccharomyces cerevisiae* wild-type yeast strain, BY4741.

### AtHMA4::GFP localization

To localise AtHMA4, a C-ter fusion of the different forms were also cloned in the pYES2 vector and expressed in the wild-type yeast strain BY4741. By Western blot using anti-EGFP horseradish peroxidase antibodies, the expression of the different chimeric proteins were controlled (Fig. 3A). The fusion proteins were observed in the microsomal fraction. Only one hybridization band which is in the range of the theoretical molecular masses of the fusion proteins (150-155 kDa) was found. No signal was observed with the microsomal fraction when the empty vector pYES2 was used, nor with AtHMA4 without EGFP, or in the soluble fraction. Under confocal microscopy, AtHMA4::EGFP appeared as a punctuated staining, typical of vesicles containing membrane proteins from the late Golgi apparatus, a pattern similar to the one described for CCC2 from *S. cerevisiae* and *Candida albicans,* respectively (Yuan *et al.,* 1997; Weissman *et al.,* 2002) (Fig. 3B). A similar pattern was observed with the 3 other forms of Athma4::EGFP. In contrast, cells transformed with EGFP alone presented a diffused cytoplasmic fluorescence.

### Cd tolerance

Drop-test experiments showed that the expression of AtHMA4 was able to increase the tolerance up to 150 µM Cd (Fig. 4A). In contrast, expression of the mutant forms *Athma4as* and *Athma4*Δ*His* did not change the wild-type phenotype (Fig. 4A). As previously reported, the *ycf1* strain was extremely sensitive to Cd (Fig. 4A) (Li *et al.,* 1996; Mason and Michaelis, 2002). When the *ycf1* strain was transformed with *AtHMA4,* the tolerance to Cd was restored to the level of the wild-type (Fig. 4A). This result shows that AtHMA4 functionally complements the *ycf1* defective strain. The two truncated forms Athma4as and Athma4ΔHis were unable to complement the *ycf1* mutant strain (Fig. 4A). To test whether increased tolerance to Cd and *ycf1* phenotypic complementation was due to transport or solely to a chelation phenomenon, a D401 A mutant of AtHMA4 (*Athma4D401A*) was generated. Asp-401 belongs to the strictly conserved motif in all P-ATPases, DKTGT, and is predicted to be the invariant residue phosphorylated in the activated enzyme. Thus the transport function must be impaired in the Athma4D401 A mutant. Indeed, this mutant did not present any increased tolerance to Cd nor complementation of the *ycf1* mutant strain (Fig. 4A). This experiment clearly demonstrates that the detoxification process of yeast cells by AtHMA4 is grounded to a transport function. Liquid culture experiments carried out in the same range of cadmium concentrations used in drop-tests confirmed the results obtained on plates. Similar experiments were carried out at 70 µM cadmium but in the presence of 5 mM buthionine sulfoximine (BSO), a known inhibitor of the γ-glutamylcysteine synthase (Griffith and Meister, 1979). In this case, the *ycf1* strain growth was completely inhibited while wild-type-pYES2 and *ycf1 - YCF1* strains grew slightly (Fig. 4B). Interestingly, the strain expressing AtHMA4 grew at the same rate in the presence or absence of BSO (Fig. 4B). This result shows that, contrary to YCF1, AtHMA4 does not need a glutathione S-conjugated form of the metal to detoxify Cd.

AtHMA4 expression in the yeast *S. cerevisiae* induces an increased tolerance to Cd (Fig. 4A) as previously described by Mills *et al.* (2003). In the presence of 150 µM Cd, the growth of the wild-type strain was rapidly impaired, whereas *AtHMA4*-transformants were still able to grow. It was no more the case when Asp-401 was replaced by Ala in AtHMA4. Asp-401 belongs to the phosphorylation consensus domain DKTGT and such mutation impairs for the transport function of P-type ATPases (Moller *et al.,* 1996). As expected, AtHMA4D401A does no longer confer an increased tolerance to Cd nor a phenotypic complementation of the *ycf1* mutant strain. This observation invalidates a chelation-based detoxification process and demonstrates a strong coupling between metal tolerance conferred by AtHMA4 and hydrolysis. In *S. cerevisiae* yeast cells, the importance of YCF1 in the Cd detoxification process has been clearly demonstrated (Li *et al.,* 1997). This protein, a member of the ATP-binding cassette family, is involved in the transport of glutathione S-conjugated Cd to the vacuole. In accordance with this activity is that the sensitivity of the *ycf1* mutant strain to Cd is strongly enhanced. Interestingly, the instant experiments demonstrate that AtHMA4 expression was able to neutralise the extrasensitivity to Cd of the defective strain. As expected, in the presence of cadmium and BSO, an inhibitor of γ-glutamylcysteine synthase (Griffith and Meister, 1979), the growth of the wild-type yeast was largely inhibited while the *ycf1* defective strain did not grow (Fig. 4B). In both strains, AtHMA4 expression restored the growth capability, demonstrating that AtHMA4 was able to preserve the yeast cells from Cd poisoning in a non GS₂-conjugated form.

### Pb / Zn / Co tolerance

Similar experiments to those described in the previous section were performed with the 3 other metals (Pb, Zn and Co) that AtHMA4 was predicted to transport. Figure 5 presents the results obtained in the presence of Pb acetate supplied in solid medium. For AtHMA4-transformants and vector-only transformants, the same capabilities of resistance were observed, up to a concentration of about 20 mM Pb. As recently published by Song *et al.* (2003), it was also observed that the *ycf1* mutant strain was highly sensitive to Pb. When AtHMA4 was expressed in the *ycf1* mutant context, it was able to partially restore the growth capability of the mutant strain while the empty vector was not able to (Fig. 5).

A series of drop-test experiments was also carried out in presence of a range of Zn concentrations (Fig. 6). AtHMA4-transformants were slightly more resistant to Zn, up to a concentration of 25 mM, than the wild-type strain. Such increased tolerance was not observed for strains expressing Athma4as and Athma4ΔHis. Interestingly, AtHMA4 was also able to rescue *zrc*1, a deletion mutant of the zinc vacuolar transporter belonging to the CDF family. As already described, this mutant strain was extremely sensitive to Zn (MacDiarmid *et al.,* 2003). AtHMA4-transformants of this strain, but not Athma4as nor Athma4ΔHis ones, exhibited a wild-type resistance to Zn.

While AtHMA4 was able to confer a tolerance to Cd, Pb and Zn concentrations, such a phenomenon was not observed in the presence of Co. The *cot1* mutant, defective for the corresponding CDF vacuolar transporter, is known to be very sensitive to Co (Conklin *et al.,* 1992). However, AtHMA4 expression in this mutant strain was unable to restore the Co tolerance.

Other metals were also screened (Ag, Cu, Ni and Fe) but no change in tolerance to these metals was observed when AtHMA4 was expressed, which is in agreement with predictions (Argüello, 2003).

Recently, it has been reported that YCF1 also participates in resistance to Pb toxicity (Song *et al.,* 2003). While AtHMA4 expressed in the *S. cerevisiae* wild-type strain did not enhance Pb resistance, it was able to restore Pb tolerance in the *ycf1* strain (Fig. 5). The instant experiments are the first demonstration of a plant P_{1B}-ATPase conferring Pb detoxification properties. Among P_{1B}-ATPases, only ZntA from *E. coli* was demonstrated to confer a tolerance to Pb (Rensing *et al.,* 1997 and 1998). Until now, NtCBP4, which belongs to the cyclic nucleotide-gated ion channel (CNGC) family, was the only example of a plant transporter involved in Pb tolerance. The overexpression of this transporter induces Pb accumulation and hypersensitivity (Arazi and al., 1999). In contrast to this hypersensitivity response, a truncated form, deleted from the C-terminal part, induced an improved tolerance to Pb (Sunkar *et al.,* 2000). The *Arabidopsis* homologue of this tobacco protein, AtCNGC1, exhibited a similar function (Sunkar *et al.,* 2000).

Besides Cd and Pb, AtHMA4 also confers an increased Zn tolerance (up to 25 mM) and a partial complementation of the defective mutant strain *zrc1* (Fig. 6). This last observation is in contradiction with previous results from Mills *et al.* (2003) who failed to observe any change in Zn resistance for this yeast strain transformed with *AtHMA4.* On the other hand, these authors observed a functional complementation of *zntA,* an *E. coli* strain defective for the Zn/Cd P-type ATPase efflux pump. The different experimental procedures and / or the range of Zn concentrations used could explain these discrepancies.

Finally, the ability of AtHMA4 to confer Co tolerance was investigated. AtHMA4 was found unable to induce an increased tolerance nor to restore growth of the yeast defective mutant *cot1* (a Co transporter belonging to the CDF family; Conklin *et al.,* 1992). Among the CPx-ATPases, CadA from *Bacillus subtilis* confers a moderate tolerance to Co (Gaballa and Helmann, 2003). It is worthy to note that CadA possesses in its N-terminal part the CxxC motif, which could be the binding site of Co, while such a motif is absent in AtHMA4.

### Cadmium accumulation (detoxification process in yeast)

After a 48 h period in the presence of 40 or 80 µM Cd, the Cd contents in the wild-type strain and the complemented strains were determined by ICP-AES. The level of Cd accumulated by the yeast was found to be approximately similar for the pYES2-only transformants and the pYES2-YCF1-complemented *ycf1* (Fig. 7). When AtHMA4 was expressed in the wild-type strain, the Cd content was drastically diminished compared to the level found in pYES2-only transformants (a fall of 50 and 62 % of the Cd content for yeast grown in the presence of 40 and 80 µM Cd, respectively). When AtHMA4 was introduced into the *ycf1* context, the fall in Cd content of the yeast cells was even more pronounced (80 and 83 % compared to *ycf1,* for yeast grown in the presence of 40 and 80 µM Cd respectively).

To limit Cd toxicity, AtHMA4 could participate in i) Cd efflux processes at the yeast plasma membrane ii) Cd transport to the vacuole iii) Cd chelation aim to the large polyhistidine motif at the C-terminal part of the protein. The results obtained with either *AtHAM4D401A* and the spliced form of AtHMA4 let us to turn down this last hypothesis. Indeed, a simple chelation process seems unlikely since these forms of the protein exhibit the polyhistidine stretch but failed to modify yeast Cd tolerance and content. The deletion in the alternative spliced form is located in the central part of the protein corresponding to the large cytosolic loop, near the strongly conserved motifs MLTGDN and GDGVNDAP. This last motif is part of the putative hinge domain (Scarborough, 2000; Xu *et al.,* 2002). Such deletion of 28 residues probably induces large perturbations in the conformational changes occurring during the catalytic process. All these results suggest that AtHMA4 is involved in an active process of Cd translocation. A Cd transport to the vacuole, mimetic to YCF1 function, would lead to an equal or even increased Cd content in the yeast cells expressing AtHMA4, relative to the wild-type strain. On the contrary, the determination of metal content indicated a drastic decrease in the Cd content in AtHMA4-transformed yeast cells compared to that in the wild-type or YCF1-transformed strains (Fig. 7). These results strongly argue for a role of AtHMA4 in Cd efflux from the yeast cell.

The decrease in the Cd content of yeast strains expressing AtHMA4 suggests a plasma membrane location of the protein which would allow an efflux of the toxic towards the external medium. However, fluorescence imaging of the AtHMA4::EGFP did not detect high levels of the protein at the plasma membrane nor at the vacuolar membrane (Fig. 3B). The punctuated staining pattern observed appears typical of vesicles from the late Golgi which traffic to the plasma membrane, as previously described (Yuan *et al,* 1997; Nothwehr *et al.,* 2000; Weissman *et al.,* 2002). Thus, we propose that AtHMA4participates in Cd loading in late Golgi vesicles driving Cd efflux at the yeast plasma membrane.

Finally, the AtHMA4ΔHis form of the protein was found non functional in metal tolerance experiments, suggesting that the polyhistidine motif is essential in the binding and / or translocation of metal. In Haemophilus, the His-rich domain in the N-ter part of the Cu,Zn superoxide dismutase has been shown to initially bind the metals to be delivered to the active site (Battistoni *et al.* (2001). The polyhistidine stretch at the C-terminal part of AtHMA4 could play a similar role of "self chaperone".

### Tissue-specific expression of AtHMA4 in Arabidopsis

The level of expression of *AtHMA4* in various organs of Ws ecotype was investigated by RT-PCR with part of the *ACTIN8* gene as a control. A 442 bp fragment in the full version of the cDNA and a 358 bp product in the case of the *Athma4as* transcript were amplified. The *AtHMA4* transcript was detected in all tissues analysed with higher expression levels in roots, stems and flowers (Fig. 8). Expression of *Athma4as* was observed in all organs studied though to a lesser extent compared to *AtHMA4.*

### T-DNA insertional mutant study

In the line Rm396, a T-DNA is inserted in the 3^{rd} intron (Fig. 9A). Southern hybridization with a probe corresponding to a fragment of the *GUS* gene showed that this insertion was unique; PCR analysis, using primers designed on *AtHMA4* (9HMA4 : 5'-CCATTAAAAGGCCTAGGATCGACATC-3' (SEQ ID NO:21)) and the T-DNA, demonstrated that the mutant line Rm396 was homozygote for the insertion. The absence of an *AtHMA4* transcript was controlled by RT-PCR, using 3 different oligonucleotide pairs positioned upstream, downstream and on both sides of the insertion, respectively. As expected, on wild-type Ws RNA extract, two amplification fragments of 708 bp and 574 bp were observed using 1HMA4 (SEQ ID NO:7)/ Rev18HMA4 (SEQ ID NO:14) and 10HMA4 (SEQ ID NO:15)/ Rev8HMA4 (SEQ ID NO:16) primer pairs, respectively. These fragments were absent when Rm396 RNA extract were used (Fig. 9B, middle and lower parts). The deletion of 7 of 9 coding exons of the *AtHMA4* gene in the Rm396 line makes it likely that AtHMA4 is non functional in this mutant.

### GUS expression under standard conditions

Interestingly, the Rm396 mutant presents a translational fusion of the *GUS* gene with the two first exons of *AtHMA4.* Figure 9B (upper part) shows a RT-PCR using a primer pair located upstream of the insertion. In this case, the reverse transcription reaction was realised with the gene specific primer Rev6HMA4 (SEQ ID NO:1). An amplification fragment was observed with Ws and Rm396 RNA, confirming the existence of a transcript corresponding to the first two exons of *AtHMA4* and the *GUS* gene. This fusion allows detection of GUS activity under the control of the *AtHMA4* promoter. GUS staining was observed in roots, around the phloem and xylem vessels, whatever the state of development of the plant (from 4 days after germination until 8 weeks-old plant) (Fig. 10a). At the level of the roots, GUS expression was particularly intense in pericycle and cambium cells (Fig. 10b). A GUS staining was also observed in the vascular tissues, under the shoot apical meristem, in the stamens and at the bases of cauline leaves (Fig. 10c-e). These results are in good agreement with those obtained by RT-PCR (Fig. 8).

### GUS expression in the presence of metals

When plants were exposed for 24 h to Zn concentrations up to 1 mM, a rise in the expression level of *AtHMA4* was observed in the roots (Fig. 11A). Such an up-regulation of the expression level of *AtHMA4* by Zn has been reported by Mills *et al.* (2003) using RT-PCR with RNA extracts from the Col-0 ecotype. Depending on the Zn concentration applied, a change in the GUS staining intensity and pattern was observed (Fig. 11B). Strikingly, when plants were grown at a high Zn concentration (1 mM), the GUS staining was strictly limited to the elongation zone of the roots (Fig. 11C). In contrast with Zn, Cd and Pb did not change the expression profile whatever the concentrations used, as probed by RT-PCR and GUS staining (data not shown). These last results disagree with a down-regulation of the expression of AtHMA4 by Cd described in Col-0 (Mills *et al.,* 2003).

### Rm396 phenotypic characterization

Since Rm396 was characterized as a knock-out mutant for *AtHMA4,* it was searched for a phenotype associated with metal uptake or metal toxicity. Young plants of Ws ecotype and line Rm396 were grown *in vitro* in the presence of a range of metal concentrations from starvation to toxicity (Cd, Zn, Co, Pb, Cu, Ni, Fe). No significant differences were observed in germination levels and plant development between the wild-type and the mutant line whatever the metals studied (data not shown). Additionally, the analysis of phenotypic characters linked to Cd toxicity (decrease in fresh weight, leaf chlorosis and Cd accumulation in roots and leaves) gave no significant differences between the wild-type and the Rm396 line.

### AtHMA4 expression in Arabidopsis

By RT-PCR, it has been shown that *AtHMA4* is expressed in all major tissues in the Ws ecotype (Fig. 8), in accordance with observations in the Col-0 ecotype. The expression pattern of AtHMA4 was studied in details by GUS staining, using the T-DNA insertional mutant from the INRA Versailles collection. GUS staining was mostly observed in roots in the region of the pericycle and cambium cells (Fig. 10). GUS staining was also associated with vascular tissues located near the shoot apical meristem, the stamens and at the bases of cauline leaves. Such localization strongly suggests that AtHMA4 could be involved in substrate transfer to or from the vascular tissues.

The presence of an alternative spliced form of the *AtHMA4* transcript, specific to the Ws ecotype, leading to a 84 bp deletion and keeping the reading frame (Fig. 2) was observed. This is the first observation of an alternative splicing for a transcript of a plant transporter. By RT-PCR, this alternative spliced form was found at a lower amount than the full-size one in all tissues studied. Several animal genes have been identified that have tissue or developmental stage-specific alternative splicing (for a review see Green, 1991). In the case of the P_{1B}-ATPases, and notably both human genes *ATP7A* (Menkes protein) and *ATP7B* (Wilson protein), they have been found in different alternative spliced forms which could account for the different subcellular distributions of these two proteins (Yang *et al.,* 1997; Qi and Byers, 1998). In contrast, in plants, alternative splicing has been rarely documented but some results indicate that it has an important role. Rubisco activase represents one of the best-known examples in which alternative splicing allows a fine-tuning of the light regulation of the enzyme (Wernecke *et al.,* 1989). A second example is the *FCA* transcript alternative splicing which is involved in flowering regulation (Macknight *et al*., 1997 and 2002). Despite different treatments and experiments have been conducted with a variety of heavy metals, we failed to observe a regulation of this alternative spliced form *in planta* and a functional role in yeast strains. However, the view cannot be excluded that this truncated protein plays a physiological role apart metal detoxification.

Since AtHMA4 is a putative Zn/Cd/Pb/Co transporter, the effects of such metals on its expression level and patterning were analysed using RT-PCR experiments and GUS staining. *AtHMA4* expression was up-regulated in roots by Zn for a concentration range up to 1 mM (Fig. 11), in accordance with RT-PCR experiments in the present study and Mills *et al.* (2003). At the higher Zn concentration (1 mM), a strong GUS staining was observed but restricted to the root elongation region. Strikingly, RT-PCR and GUS staining experiments did not indicate any regulation of *AtHMA4* expression by Cd or Pb, which is in contrast to previous results from Mills *et al*.(2003). The known differences in Cd tolerance between Ws and Col-0 ecotypes, and in the expression profiles found in Ws and Col-0, may be involved in these divergent observations. Furthermore, these studies were conducted with different salts of Cd (CdCl₂ in this work *vs*. CdSO₄ in other work), and the associated anion may create the different results.

A defective mutant of AtHMA4 does not exhibit any specific phenotype under normal or metal excess conditions and such an absence of phenotype could result from the presence of other transporters, belonging to the same (such as AtHMA2 and AtHMA3) or to other protein families, and supporting redundant functions. This is illustrated in the yeast since some AtHMA4 properties were only observed in a mutant context.

### Overexpression of AtHMA4

To test the role of *AtHMA4* in *Arabidopsis,* plants ectopically overexpressing this gene were generated. The AtHMA4 cDNA was cloned under the strong constitutive CaMV35S promoter and introduced in *Arabidopsis* through *Agrobacterium tumefaciens*-mediated transformation. Two independent lines with elevated *AtHMA4* mRNA levels (Fig. 12A) carrying single T-DNA insertion were selected and self-crossed to obtain stable homozygous lines. The Zn content of shoot from overexpressing plants, hydroponically grown on nutrient solution (3 µM Zn) was measured by ICP-AES. *AtHMA4* leaves from overexpressing plants accumulated 61 % more Zn than the control (Fig. 12B). Both overexpressing lines displayed Cd and Zn increased tolerance as assayed by root growth measurements (Fig. 12C and D). On control solid medium (Zn 3 µM), root lengths of the overexpressing plants were not statistically different from the one of the wild-type. However, on media containing high Zn concentrations (50, 200 µM) or Cd (10, 40 µM), the root lengths from overexpressing lines were greater by 35, 154, 43 and 123 % compared to wild-type roots, respectively.

Despite it was failed to find a phenotype for the knock-out mutant, overexpression of AtHMA4 *in planta* lead to an increased tolerance to Zn and Cd (Fig. 12 D), as already observed in the yeast. This increased tolerance was particularly obvious at the level of the root development during a Zn or Cd treatment (as an example, the root was two times longer for the overexpressing lines in comparison to the wild-type plants in presence of Cd 40 µM). When grown in control conditions, the overexpressing lines did not present longer roots, but the Zn content was 61 % higher than the one of the wild-type.

Altogether these observations strongly suggest that AtHMA4 participates in the loading of heavy metal in the xylem, allowing their translocation to the shoot.

The same may be observed for AtHMA1-3.

### EXAMPLE 3: COUPLING OF HMA3 AND HMA4

AtHMA3 belongs to the same P_{1B} sub-group of ATPases as AtHMA4. This enzyme is not present in all the wild-type *Arabidosis thaliana* ecotypes. In particular, AtHMA3 is expressed in the Wassilewskija ecotype, whereas it is absent in the Columbia ecotype.

By expressing this enzyme heterologously in yeast, the inventors have shown that AtHMA3 transports cadmium, cobalt and lead, but probably not zinc. AtHMA3 is localized at the vacuole and carries out sequestration of metals in this organite, in yeast (Gravot et al, FEBS Lett. 2004).

In plants, AtHMA3 is also localised at the vacuole membrane, and detoxifies cells by sequestrating toxic metals in the vacuole. In genetically engineered plants that overexpress both HMA3 and HMA4, these two enzymes act synergistically: HMA4 enhances translocation of heavy metals from the roots to the upper parts of the plant, whereas HMA3 favours vacuolar sequestration. As a result, the plants tolerate toxic concentration of heavy metals during a longer period, hence enabling a stronger extraction of said metals from contaminated soils.

### LITERATURE CITED

Arazi T, Sunkar R, Kaplan B and Fromm H (1999) A tobacco plasma membrane calmodulin-binding transporter confers Ni2+ tolerance and Pb2+ hypersensitivity in transgenic plants. Plant J 20:171-182
Argüello JM (2003) Identification of ion-selectivity determinants in heavy-metal transport P1B-type ATPase. J Membrane Biol 195:93-108
Axelsen KB, Palmgren MG (2001) Inventory of the superfamily of P-type ion pumps in Arabidopsis. Plant Physiol 126:696-706
Bassett Jr DE, Boguski MS and Hieter P (1996) Yeast genes and human disease. Nature 379:589-590
Battistoni A, Pacello F, Mazzetti AP, Capo C, Kroll JS, Langford PR, Sansone A, Donnarumma G, Valenti P and Rotilio G (2001) A histidine-rich metal binding domain at the N terminus of Cu,Zn-superoxide dismutases from pathogenic bacteria. J Biol Chem 276:30315-30325
Baxter I, Tchieu J, Sussman MR, Boutry M, Palmgren MG, Gribskov M, Harper JF and Axelsen KB (2003) Genomic comparison of P-type ATPase ion pumps in Arabidopsis and rice. Plant Physiol 132:618-628
Catty P, de Kerchove d'Exaerde A and Goffeau A (1997) The complete inventory of the yeast Saccharomyces cerevisiae P-type transport ATPases. FEBS Lett 409:325-332
Clemens S (2001) Molecular mechanisms of plant metal tolerance and homeostasis. Planta 212:475-486
Clough SJ and Bent AF (1998) Floral dip: a simplified method for Agrobacterium-mediated transformation of Arabidopsis thaliana. Plant J 16:735-743
Conklin DS, McMaster JA, Culbertson MR and Kung C (1992) COT1, a gene involved in cobalt accumulation in Saccharomyces cerevisiae. Mol Cell Biol 12:3678-3688
Gravot, A., A. Lieutaud, et al. (2004). "AtHMA3, a plant P1B-ATPase, functions as a Cd/Pb transporter in yeast." FEBS Lett 561(1-3): 22-8.
Fu D, Beeler TJ and Dunn TM (1995) Sequence, mapping and disruption of CCC2, a gene that cross-complements the Ca(2+)-sensitive phenotype of csg1 mutants and encodes a P-type ATPase belonging to the Cu(2+)-ATPase subfamily. Yeast 11:283-292
Gaballa A and Helmann JD (2003) Bacillus subtilis CPx-type ATPases: characterization of Cd, Zn, Co and Cu efflux systems. Biometals 16:497-505
Green MR (1991) Biochemical mechanisms of constitutive and regulated pre-mRNA splicing. Annu Rev Cell Biol 7:559-599
Griffith OW and Meister A (1979) Potent and specific inhibition of gluthatione synthesis by buthionine sulfoximine (S-n-butylhomocysteine sulphoximine). J Biol Chem 254:7558-7560
Hirayama T, Kieber JJ, Hirayama N, Kogan M, Guzman P, Nourizadeh S, Alonso JM, Dailey W and Dancis A (1999) RESPONSIVE-TO-ANTAGONIST1, a Menkes/Wilson disease-related copper transporter, is required for ethylene signaling in Arabidopsis. Cell 97:383-93
Hou Z and Mitra B (2003) The metal specificity and selectivity of ZntA from Escherichia coli using the acylphosphate intermediate. J Biol Chem 278:28455-28461 Inesi G (1985) Mechanism of calcium transport. Annu. Rev. Physiol 47:573-601 Jefferson RA, Kavanagh TA and Bevan MW (1987) GUS fusions: β-glucuronidase as a sensitive and versatile gene fusion marker in higher plants. EMBO J 6:3901-3907 Lebrun M, Audurier A and Cossart P (1994a) Plasmid-borne cadmium resistance genes in Listeria monocytogenes are similar to cadA and cadC of Staphylococcus aureus and are inducible by cadmium. J Bacteriol 176:3040-3048
Lebrun M, Audurier A and Cossart P (1994b) Plasmid-borne cadmium resistance genes in Listeria monocytogenes are present on Tn5422, a novel transposon closely related to Tn917. J Bacteriol 176:3049-3061
Lee J. et al., Plant Phsyiol., 2003, 133, 589-596
Li ZS, Szczypka M, Lu YP, Thiele DJ and Rea PA (1996) The yeast cadmium factor protein (YCF1) is a vacuolar glutathione S-conjugate pump. J Biol Chem 271:6509-6517
Li ZS, Lu YP, Zhen RG, Szczypka M, Thiele DJ and Rea PA (1997) A new pathway for vacuolar cadmium sequestration in Saccharomyces cerevisiae: YCF1-catalyzed transport of bis(glutathionato)cadmium. Proc Natl Acad Sci USA 94: 42-47 MacDiarmid CW, Milanick MA and Eide DJ (2003) Induction of the ZRCI metal tolerance gene in zinc-limited yeast confers resistance to zinc shock. J Biol Chem 278:15065-15072
Macknight R, Bancroft I, Page T, Lister C, Schmidt R, Love K, Westphal L, Murphy G, Sherson S, Cobbett C and Dean C (1997) FCA, a gene controlling flowering time in Arabidopsis, encodes a protein containing RNA-binding domains. Cell 89:737-745 Macknight R, Duroux M, Laurie R, Dijkwel P, Simpson G and Dean C (2002) Functional significance of the alternative transcript processing of the Arabidopsis floral promoter FCA. Plant Cell 14:877-888
Mason DL and Michaelis S (2002) Requirement of the N-terminal extension for vacuolar trafficking and transport activity of yeast Ycflp, an ATP-binding cassette transporter. Mol Biol Cell 13:4443-4455
Mills RF, Krijger GC, Baccarini PJ, Hall JL and Williams LE (2003) Functional expression of AtHMA4, a P1B-type ATPase of the Zn/Co/Cd/Pb subclass. Plant J 35:164-176
Moller JV, Juul B and LeMaire M (1996) Structural organization, ion transport, and energy transduction of P-type ATPases. Biochim Biophys Acta 1286:1-51
Murashige T and Skoog F (1962) A revised medium for rapid growth and bio assays with tobacco tissue cultures. Physiol Plant 15:473-497
Nothwehr SF, Ha S-A and Bruinsma P (2000) Sorting of yeast membrane proteins into an endosome-to-Golgi pathway involves direct interaction of their cytosolic domains with Vps35p. J Cell Biol 151:297-310
Qi M and Byers PH (1998) Constitutive skipping of alternatively spliced exon 10 in the ATP7A gene abolishes Golgi localization of the Menkes protein and produces the occipital horn syndrome. Hum Mol Genet 7:465-469
Ran MR, Kirchrath L and Hollenberg CP (1994) A putative P-type Cu(2+)-transporting ATPase gene on chromosome II of Saccharomyces cerevisiae. Yeast 10:1217-1225
Rensing C, Mitra B and Rosen BP (1997) The zntA gene of Escherichia coli encodes a Zn (II)-translocating P-type ATPase. Proc Natl Acad Sci USA 94:14326-14331 Rensing C, Sun Y, Mitra B and Rosen BP (1998) Pb(II)-translocating P-type ATPases. J Biol Chem 273:32614-32617
Sambrook J, Fritsch EF and Maniatis T (1989) Molecular cloning: A Laboratory Manual, Ed 2. Cold Spring Harbor Laboratory Press, Cold Spring Harbor, NY Scarborough GE (2000) Crystallisation, structure and dynamics of the proton-translocating P-type ATPase. J Exp Biol 203:147-154
Shikanai T, Müller-Moulé P, Munekage Y, Niyogi KK and Pilon M (2003) PAA1, a P-type ATPase of Arabidopsis, functions in copper transport in chloroplasts. Plant Cell 15:1333-1346.
Solioz M and Vulpe C (1996) CPx-type ATPases: a class of P-type ATPases that pump heavy metals. Trends Biochem Sci 21:237-241
Song W-Y, Sohn EJ, Martinoia E, Lee YJ, Yang Y-Y, Jasinski M, Forestier C, Hwang I and Lee Y (2003) Engineering tolerance and accumulation of lead and cadmium in transgenic plants. Nature Biotechnol 21:914-919
Sunkar R, Kaplan B, Bouché N, Arazi T, Dolev D, Talke IN, Maathuis FJ, Sanders D, Bouchez D and Fromm H (2000) Expression of a truncated tobacco NtCBP4 channel in transgenic plants and disruption of the homologous Arabidopsis CNGCI gene confer Pb2+ tolerance. Plant J 24:533-542
Tsai K-J, Lin Y-F, Wong MD, Yang H H-C, Fu H-L and Rosen BP (2002) Membrane topology of the p1258 CadA Cd(II)/Pb(II)/Zn(II)-translocating P-type ATPase. J Bioenerg Biomembr 34:147-156
Verwoerd TC, Dekker BM and Hoekema (1989) A small-scale procedure for the rapid isolation of plant RNAs. Nucleic Acids Res 17:2362
Weissman Z, Shemer R and Komitzer D (2002) Deletion of the copper transporter CaCCC2 reveals two distinct pathways for iron acquisition in Candida albicans. Mol Microbiol 44:1551-1560
Werneke JM, Chatfield JM and Ogren WL (1989) Alternative mRNA splicing generates the two ribulosebisphosphate carboxylase oxygenase activase polypeptides in spinach and Arabidopsis. Plant Cell 1:8115-825
Williams LE, Pittman JK and Hall JL (2000) Emerging mechanisms for heavy metal transport in plants. Biochim Biophys Acta 1465:104-126
Woeste KE and Kieber JJ (2000) A strong loss-of function mutation in RAN1 results in constitutive activation of the ethylene response pathway as well as a rosette-lethal phenotype. Plant Cell. 12:443-55.
Xu C, Rice WJ, He W and Stokes DL (2002) A structural model for the catalytic cycle of Ca2+-ATPase. J Mol Biol 316:201-211
Yang X-L, Miura N, Kawarada Y, Terada K, Petrukhin K, Gilliam T and Sugiyama T (1997) Two forms of Wilson disease protein produced by alternative splicing are localized in distinct cellular compartments. Biochem J 326:897-902
Yuan DS, Dancis A and Klausner RD (1997) Restriction of copper export in Saccharomyces cerevisiae to a late Golgi or post-Golgi compartment in the secretory pathway. J Biol Chem 272:25787-25793

## Claims

1. Genetically modified plants, **characterized in that** they overexpress one or more than one copy of at least a sequence encoding a higher plant-origin P_{1B}-type ATPase of the Zn²⁺/Co²⁺/Cd²⁺/Pb²⁺ subclass and that they are able to accumulate heavy metals and translocate them to the shoots.

2. Genetically modified plants according to claim 1, **characterized in that** said P_{1B}-type ATPase is selected from the group consisting of heavy metal ATPase HMA1, HMA2, HMA3 and HMA4.

3. Genetically modified plants according to claim 2, **characterized in that** said P_{1B}-type ATPase is selected from the group consisting of endogenous ATPases HMA1, HMA2, HMA3 and HMA4.

4. Genetically modified plants according to claim 2, **characterized in that** said P_{1B}-type ATPase is selected from the group consisting of heavy metal ATPases HMA1, HMA2, HMA3 and HMA4 of *Arabidopsis thaliana.*

5. Genetically modified plants according to any of claims 1 to claim 4, **characterized in that** said genetically modified plants overexpress one or more than one copy of at least two different sequences encoding two different higher plant-origin P_{1B}-type ATPases of the Zn²⁺/Co²⁺/Cd²⁺/Pb²⁺ subclass.

6. Genetically modified plants according to claim 5, **characterized in that** said genetically modified plants overexpress one or more than one copy of at least HMA3 and HMA4.

7. Genetically modified plants according to any of claims 1 to claim 6, **characterized in that** they overexpress one or more than one copy of a sequence encoding at least an eukaryotic P_{1B}-type ATPase of the Zn²⁺/Co²⁺/Cd²⁺/Pb²⁺ subclass and at least another sequence selected among sequences encoding (1) an enzyme involved in metal chelation or (2) another metal transporter such as YCF1 or other ABC transporters.

8. Genetically modified plants according to claim 7, **characterized in that** said enzyme involved in metal chelation is selected from the group consisting of phytochelatin synthase, glutathion synthetase and gamma-glutamylcystein synthase.

9. Recombinant vector able to transform plants, **characterized in that** said vector includes one or more than one copy of at least a sequence encoding a higher plant-origin P_{1B}-type ATPase of the Zn²⁺/Co²⁺/Cd²⁺/Pb²⁺ subclass, wherein said ATPase is able to accumulate heavy metals and translocate them to the shoots of a plant when expressed in said plant.

10. Recombinant vector according to claim 9, wherein said ATPase is selected from the group consisting of ATPases HMA1, HMA2, HMA3 and HMA4.

11. Recombinant vector according to claim 9 or claim 10, **characterized in that** said coding sequences are operably linked to and under the regulatory control of a plant-expressible transcription and translation regulatory sequence, such as a plant specific promoter.

12. Recombinant vector according to any of claims 9 to 11, **characterized in that** it comprises a first sequence encoding HMA3, and a second sequence encoding HMA4.

13. A set of recombinant vectors according to any of claims 9 to 11, **characterized in that** it comprises at least a first vector encoding HMA3 and a second vector encoding HMA4.

14. Genetically modified plants overexpressing at least one higher plant-origin P_{1B}-type ATPase of the Zn²⁺/Co²⁺/Cd²⁺/Pb²⁺ subclass, obtainable by transforming plants with a recombinant vector according to any one of claims 9 to 12, or with a set of vectors according to claim 13.

15. Genetically modified plants, as defined in any one of claims 1 to 8 and 14, **characterized in that** said plants are selected in the group consisting of *Brassica juncea, Poplar, Nicotiana tabacum.*

16. Genetically transformed plant cells overexpressing at least one higher plant-origin P_{1B}-type ATPase of the Zn²⁺/Co²⁺/Cd²⁺/Pb²⁺ subclass, obtainable by transforming plant cells with a recombinant vector according to any of claims 9 to 12, or with a set of vectors according to claim 13.

17. Use of genetically modified plants according to any one of claims 1 to 8, 14 or 15, for phytoextraction of Zn, Co, Cd or Pb, from a contaminated environment.

18. Use of genetically modified plants according to claim 6, for phytoextraction of Co, Cd or Pb, from a contaminated environment.

19. Method of producing genetically modified plants according to claims 1 to 8, 14 or 15 which overexpress, at least a P_{1B}-type ATPase of the Zn²⁺/Co²⁺/Cd²⁺/Pb²⁺ subclass, said method comprising:
- preparing at least one recombinant vector(s) according to any of claims 9 to 12, and
- introducing said at least one recominant vector(s) into a plant cell or plant tissue to produce a genetically modified plant cell or a genetically modified plant tissue.

20. Method of phytoremediation of heavy metals from soil, **characterized in that** it includes:
- a step of planting genetically modified plants according to any one of claims 1 to 8, 14 or 15, in an area containing soil contaminated with at least one heavy metal and
- collecting and removing plant tissues from said genetically modified plants at appropriate time intervals.

21. Method of phytoremediation according to claim 20, **characterized in that** it involves the extraction of at least one of the following heavy metals: Zn, Co, Cd or Pb, from soil.

22. Method of phytoremediation according to claim 20 or claim 21, **characterized in that** the entire plant is removed after it has been allowed to grow on metal-containing soil.

23. Method of phytoremediation according to claim 20 or claim 21, **characterized in that** at appropriate time intervals, metal containing tissues are removed from the plant, said plant being left alive.

24. The method of claim 23, wherein leaves and possibly branches are removed.

25. Method of phytoremediation according to claim 23 or claim 24, **characterized in that** the collected plant tissues are removed from the growing area and properly disposed, so that the metal containing tissues are not allowed to reassimilate in the soil.

26. Method of phytoremediation according to claim 23 or claim 24, **characterized in that** said heavy metals may be extracted, in the Mⁿ⁺ state, from said plant tissues.

27. Method of phytoremediation according to claim 23 or claim 24, **characterized in that** said heavy metals are extracted from ashes obtained after having burnt the collected metal containing tissues, said metal being in the M⁰ state.

## Patentansprüche

1. Genetisch modifizierte Pflanzen, **dadurch gekennzeichnet, dass** sie eine oder mehr als eine Kopie wenigstens einer Sequenz, die eine P_{1B}-Typ-ATPase der Zn²⁺/Co²⁺/Cd²⁺/Pb²⁺-Subklasse mit Herkunft von einer höheren Pflanze codiert, überexprimieren und dass sie fähig sind, Schwermetalle zu akkumulieren und sie in die Schösslinge zu translozieren.

2. Genetisch modifizierte Pflanzen nach Anspruch 1, **dadurch gekennzeichnet, dass** die P_{1B}-Typ-ATPase aus der Gruppe, bestehend aus Schwermetall-ATPasen HMA1, HMA2, HMA3 und HMA4, ausgewählt ist.

3. Genetisch modifizierte Pflanze nach Anspruch 2, **dadurch gekennzeichnet, dass** die P_{1B}-Typ-ATPase aus der Gruppe, bestehend aus endogenen ATPasen HMA1, HMA2, HMA3 und HMA4, ausgewählt ist.

4. Genetisch modifizierte Pflanzen nach Anspruch 2, **dadurch gekennzeichnet, dass** die P_{1B}-Typ-ATPase aus der Gruppe, bestehend aus Schwermetall-ATPasen HMA1, HMA2, HMA3 und HMA4 von *Arabidopsis thaliana,* ausgewählt ist.

5. Genetisch modifizierte Pflanzen nach einem der Ansprüche 1 bis 4, **dadurch gekennzeichnet , dass** die genetisch modifizierten Pflanzen eine oder mehr als eine Kopie von wenigstens zwei verschiedenen Sequenzen, die zwei verschiedene P_{1B}-Typ-ATPasen der Zn²⁺/Co²⁺/Cd²⁺/Pb²⁺-Subklasse codieren, überexprimieren.

6. Genetisch modifizierte Pflanzen nach Anspruch 5, **dadurch gekennzeichnet, dass** die genetisch modifizierten Pflanzen eine oder mehr als eine Kopie von wenigstens HMA3 und HMA4 überexprimieren.

7. Genetisch modifizierte Pflanzen nach einem der Ansprüche 1 bis 6, **dadurch gekennzeichnet, dass** sie eine oder mehr als eine Kopie einer Sequenz, die wenigstens eine eukaryotische P_{1B}-Typ-ATPase der Zn²⁺/Co²⁺/Cd²⁺/Pb²⁺-Subklasse codiert, und wenigstens einer anderen Sequenz, ausgewählt unter Sequenzen, die (1) ein Enzym, das bei der Metallchelatbildung involviert ist, oder (2) einen anderen Metalltransporter, zum Beispiel YCF1, oder andere ABC-Transporter codieren, überexprimieren.

8. Genetisch modifizierte Pflanze nach Anspruch 7, **dadurch gekennzeichnet, dass** das Enzym, das bei der Chelatbildung involviert ist, aus der Gruppe, bestehend aus Phytochelatinsynthase, Gluthathionsynthetase und gamma-Glutamylcysteinsynthase, ausgewählt ist.

9. Rekombinanter Vektor, der fähig ist, Pflanzen zu transformieren, **dadurch gekennzeichnet, dass** der Vektor eine oder mehr als eine Kopie wenigstens einer Sequenz, die eine P_{1B}-Typ-ATPase der Zn²⁺/Co²⁺/Cd²⁺/Pb²⁺-Subklasse mit einer Herkunft von einer höheren Pflanze codiert, umfasst, wobei die ATPase fähig ist, Schwermetalle zu akkumulieren und sie zu den Schösslingen einer Pflanze zu translozieren, wenn sie in der Pflanze exprimiert wird.

10. Rekombinanter Vektor nach Anspruch 9, wobei die ATPase aus der Gruppe, bestehend aus ATPasen HMA1, HMA2, HMA3 und HMA4, ausgewählt ist.

11. Rekombinanter Vektor nach Anspruch 9 oder Anspruch 10, **dadurch gekennzeichnet, dass** die codierenden Sequenzen funktionell verknüpft mit und unter der regulatorischen Kontrolle einer in Pflanzen exprimierbaren Transkriptions- und Translationsregulationssequenz, zum Beispiel ein Pflanzen-spezifischer Promotor, sind.

12. Rekombinanter Vektor nach einem der Ansprüche 9 bis 11, **dadurch gekennzeichnet , dass** er eine erste Sequenz, die HMA3 codiert, und eine zweite Sequenz, die HMA4 codiert, umfasst.

13. Satz rekombinanter Vektoren nach einem der Ansprüche 9 bis 11, **dadurch gekennzeichnet, dass** er wenigstens einen ersten Vektor, der HMA3 codiert, und einen zweiten Vektor, der HMA4 codiert, umfasst.

14. Genetisch modifizierte Pflanzen, die wenigstens eine P_{1B}-Typ-ATPase der Zn²⁺/Co²⁺/Cd²⁺/Pb²⁺-Subklasse mit Herkunft von einer höheren Pflanze überexprimieren, erhältlich durch Transformieren von Pflanzen mit einem rekombinanten Vektor nach einem der Ansprüche 9 bis 12 oder mit einem Satz von Vektoren nach Anspruch 13.

15. Genetisch modifizierte Pflanzen, wie sie in einem der Ansprüche 1 bis 8 und 14 definiert sind, **dadurch gekennzeichnet, dass** die Pflanzen aus Gruppe, bestehend aus *Brassica juncea, Populus, Nicotiana tabacum,* ausgewählt sind.

16. Genetisch transformierte Pflanzenzellen, die wenigstens eine P_{1B}-Typ-ATPase der Zn²⁺/Co²⁺/Cd²⁺/Pb²⁺-Subklasse mit Herkunft von einer höheren Pflanze überexprimieren, die durch Transformieren von Pflanzenzellen mit einem rekombinanten Vektor nach einem der Ansprüche 9 bis 12 oder mit einem Satz von Vektoren nach Anspruch 13 erhältlich sind.

17. Verwendung von genetisch modifizierten Pflanzen nach einem der Ansprüche 1 bis 8, 14 oder 15 zur Phytoextraktion von Zn, Co, Cd oder Pb aus einer kontaminierten Umgebung.

18. Verwendung von genetisch modifizierten Pflanzen nach Anspruch 6 zur Phytoextraktion von Co, Cd oder Pb aus einer kontaminierten Umgebung.

19. Verfahren zur Produktion genetisch modifizierter Pflanzen nach den Ansprüchen 1 bis 8, 14 oder 15, welche wenigstens eine P_{1B}-Typ-ATPase der Zn²+/Co²⁺/Cd²⁺/Pb²⁺-Subklasse überexprimieren, wobei das Verfahren umfasst:
- Herstellen wenigstens eines rekombinanten Vektors nach einem der Ansprüche 9 bis 12 und
- Einführen wenigstens eines rekombinanten Vektors in eine Pflanzenzelle oder ein Pflanzengewebe, um eine genetisch modifizierte Pflanzenzelle oder ein genetisch modifiziertes Pflanzengewebe zu produzieren.

20. Verfahren zur Phytoremediation von Schwermetallen aus Boden, **dadurch gekennzeichnet, dass** es umfasst:
- einen Schritt des Pflanzens genetisch modifizierter Pflanzen nach einem der Ansprüche 1 bis 8, 14 oder 15, in einem Gebiet, das Boden enthält, der mit wenigstens einem Schwermetall kontaminiert ist, und
- Sammeln und Entfernen von Pflanzengeweben aus den genetisch modifizierten Pflanzen in geeigneten Zeitintervallen.

21. Verfahren zur Phytoremediation nach Anspruch 20, **dadurch gekennzeichnet, dass** es die Extraktion wenigstens eines der folgenden Schwermetalle: Zn, Co, Cd oder Pb, aus Boden involviert.

22. Verfahren zur Phytoremediation nach Anspruch 20 oder Anspruch 21, **dadurch gekennzeichnet, dass** die ganze Pflanze entfernt wird, nachdem sie auf dem Metall-enthaltenden Boden wachsen gelassen wurde.

23. Verfahren zur Phytoremediation nach Anspruch 20 oder Anspruch 21, **dadurch gekennzeichnet, dass** in geeigneten Zeitintervallen Metall-enthaltende Gewebe aus der Pflanze entfernt werden, wobei die Pflanze am Leben gelassen wird.

24. Verfahren nach Anspruch 23, wobei Blätter und möglicherweise Äste entfernt werden.

25. Verfahren zur Phytoremediation nach Anspruch 23 oder Anspruch 24, **dadurch gekennzeichnet , dass** die gesammelten Pflanzengewebe aus dem Wachstumsgebiet entfernt und geeigneterweise entsorgt werden, so dass die Metall-enthaltenden Gewebe in dem Boden nicht reassimilieren gelassen werden.

26. Verfahren zur Phytoremediation nach Anspruch 23 oder Anspruch 24, **dadurch gekennzeichnet , dass** die Schwermetalle im Mⁿ⁺-Zustand aus den Pflanzengeweben extrahiert werden können.

27. Verfahren zur Phytoremediation nach Anspruch 23 oder Anspruch 24, **dadurch gekennzeichnet , dass** die Schwermetalle aus Aschen extrahiert werden, die erhalten werden, nachdem die gesammelten Metall-enthaltenden Gewebe verbrannt wurden, wobei das Metall im M⁰-Zustand ist.

## Revendications

1. Plantes génétiquement modifiées, **caractérisées en ce qu'**elles surexpriment une ou plus d'une copie d'au moins une séquence codant pour une ATPase de type P_{1B} d'origine végétale supérieure de la sous-classe Zn²⁺ / Co²⁺ / Cd²⁺ / Pb²⁺ et **en ce qu'**elles sont capables d'accumuler des métaux lourds et de les transloquer vers leurs parties aériennes.

2. Plantes génétiquement modifiées selon la revendication 1, **caractérisées en ce que** ladite ATPase de type P_{1B} est choisie dans le groupe comprenant les ATPases à métaux lourds HMA1, HMA2, HMA3 et HMA4.

3. Plantes génétiquement modifiées selon la revendication 2, **caractérisées en ce que** ladite ATPase de type P_{1B} est choisie dans le groupe comprenant les ATPases endogènes HMA1, HMA2, HMA3 et HMA4.

4. Plantes génétiquement modifiées selon la revendication 2, **caractérisées en ce que** ladite ATPase de type P_{1B} est choisie dans le groupe comprenant les ATPases à métaux lourds HMA1, HMA2, HMA3 et HMA4 *d'Arabidopsis thaliana.*

5. Plantes génétiquement modifiées selon l'une quelconque des revendications 1 à 4, **caractérisées en ce que** lesdites plantes génétiquement modifiées surexpriment une ou plus d'une copie d'au moins deux séquences différentes codant pour deux ATPase de type P_{1B} d'origine végétale supérieure différentes de la sous-classe Zn²⁺ / Co²⁺ /Cd²⁺ /Pb²⁺.

6. Plantes génétiquement modifiées selon la revendication 5, **caractérisées en ce que** lesdites plantes génétiquement modifiées surexpriment une ou plus d'une copie parmi au moins HMA3 et HMA4.

7. Plantes génétiquement modifiées selon l'une quelconque des revendications 1 à 6, **caractérisées en ce qu'**elles surexpriment une ou plus d'une copie d'une séquence codant pour au moins une ATPase de type P_{1B} d'eucaryote de la sous-classe Zn²⁺ / Co²⁺ / Cd²⁺ /Pb²⁺ et d'au moins une autre séquence choisie parmi les séquences codant (1) pour une enzyme impliquée dans la chélation des métaux ou (2) pour un autre transporteur de métaux tel que le YCF1 ou d'autres transporteurs ABC.

8. Plantes génétiquement modifiées selon la revendication 7, **caractérisées en ce que** ladite enzyme impliquée dans la chélation des métaux est choisie dans le groupe comprenant la phytochélatine synthase, la glutathion synthétase et la gamma-glutamylcystéine synthase.

9. Vecteur recombinant capable de transformer des plantes, **caractérisé en ce que** ledit vecteur comprend une ou plus d'une copie parmi au moins une séquence codant pour une ATPase de type P_{1B} d'origine végétale supérieure de la sous-classe Zn²⁺ / Co²⁺ / Cd²⁺ /Pb²⁺, ladite ATPase étant capable d'accumuler des métaux lourds et de les transloquer vers les parties aériennes d'une plante lorsqu'elle est exprimée dans ladite plante.

10. Vecteur recombinant selon la revendication 9, dans lequel ladite ATPase est choisie dans le groupe comprenant les ATPase HMA1, HMA2, HMA3 et HMA4.

11. Vecteur recombinant selon la revendication 9 ou la revendication 10, **caractérisé en ce que** lesdites séquences codantes sont liées de manière fonctionnelle à et sous le contrôle régulateur d'une séquence régulatrice de la transcription et de la traduction pouvant être exprimée dans une plante, telle qu'un promoteur spécifique d'une plante.

12. Vecteur recombinant selon l'une quelconque des revendications 9 à 11, **caractérisé en ce qu'**il comprend une première séquence codant pour la HMA3, et une seconde séquence codant pour la HMA4.

13. Ensemble de vecteurs recombinants selon l'une quelconque des revendications 9 à 11, **caractérisé en ce qu'**il comprend au moins un premier vecteur codant pour la HMA3 et un second vecteur codant pour la HMA4.

14. Plantes génétiquement modifiées surexprimant au moins une ATPase de type P_{1B} d'origine végétale supérieure de la sous-classe Zn²⁺ / Co²⁺ /Cd²⁺ /Pb²⁺, pouvant être obtenue en transformant des plantes à l'aide d'un vecteur recombinant selon l'une quelconque des revendications 9 à 12, ou à l'aide d'un ensemble de vecteurs selon la revendication 13.

15. Plantes génétiquement modifiées, selon l'une quelconque des revendications 1 à 8 et 14, **caractérisées en ce que** lesdites plantes sont choisies dans le groupe comprenant *Brassica juncea, Populus, Nicotiana tabacum.*

16. Cellules végétales génétiquement modifiées surexprimant au moins une ATPase de type P_{1B} d'origine végétale supérieure de la sous-classe Zn²⁺ / Co²⁺ / Cd²⁺ /Pb²⁺, pouvant être obtenues en transformant des cellules végétales à l'aide d'un vecteur recombinant selon l'une quelconque des revendications 9 à 12, ou à l'aide d'un ensemble de vecteurs selon la revendication 13.

17. Utilisation de plantes génétiquement modifiées selon l'une quelconque des revendications 1 à 8, 14 ou 15, pour la phyto-extraction du Zn, Co, Cd ou Pb, à partir d'un environnement contaminé.

18. Utilisation de plantes génétiquement modifiées selon la revendication 6, pour la phyto-extraction du Co, Cd ou Pb, à partir d'un environnement contaminé.

19. Procédé de production de plantes génétiquement modifiées selon les revendications 1 à 8, 14 ou 15, qui surexpriment au moins une ATPase de type P_{1B} de la sous-classe Zn²⁺ / Co²⁺ / Cd²⁺ / Pb²⁺, ledit procédé comprenant les étapes suivantes :
- préparer au moins un vecteur recombinant selon l'une quelconque des revendications 9 à 12, et
- introduire ledit au moins un vecteur recombinant à l'intérieur d'une cellule végétale ou de tissus végétaux afin de produire une cellule végétale génétiquement modifiée ou des tissus végétaux génétiquement modifiés.

20. Procédé de phyto-remédiation de métaux lourds à partir du sol, **caractérisé en ce qu'**il comprend :
- une étape consistant à planter des plantes génétiquement modifiées selon l'une quelconque des revendications 1 à 8, 14 ou 15, dans une zone contenant un sol contaminé par au moins un métal lourd et
- une étape consistant à collecter et à éliminer des tissus végétaux provenant desdites plantes génétiquement modifiées à des intervalles de temps appropriés.

21. Procédé de phyto-remédiation selon la revendication 20, **caractérisé en ce qu'**il comprend l'extraction d'au moins un des métaux lourds suivants : Zn, Co, Cd ou Pb, à partir du sol.

22. Procédé de phyto-remédiation selon la revendication 20 ou la revendication 21, **caractérisé en ce que** la plante entière est éliminée après qu'elle ait été laissée croître sur le sol contenant les métaux.

23. Procédé de phyto-remédiation selon la revendication 20 ou la revendication 21, **caractérisé en ce que**, à des intervalles de temps appropriés, les tissus contenant les métaux sont éliminés de la plante, ladite plante étant gardée vivante.

24. Procédé selon la revendication 23, dans lequel des feuilles et éventuellement des branches sont éliminées.

25. Procédé de phyto-remédiation selon la revendication 23 ou la revendication 24, **caractérisé en ce que** les tissus végétaux collectés sont éliminés de la zone de croissance et détruits de manière appropriée, de sorte que les tissus contenant les métaux ne sont pas admis à être réassimilés dans le sol.

26. Procédé de phyto-remédiation selon la revendication 23 ou la revendication 24, **caractérisé en ce que** lesdits métaux lourds peuvent être extraits, à l'état Mⁿ⁺, à partir desdits tissus végétaux.

27. Procédé de phyto-remédiation selon la revendication 23 ou la revendication 24, **caractérisé en ce que** lesdits métaux lourds sont extraits des cendres obtenues après avoir brûlé les tissus collectés contenant les métaux, lesdits métaux étant à l'état M⁰.


### SEQUENCE LISTING

<110> COMMISSARIAT A L'ENERGIE ATOMIQUE
   RICHAUD, Pierre
   VERRET, Frédéric
   GRAVOT, Antoine
   AUROY, Pascaline
   VAVASSEUR, Alain
<120> Genetically modified plants and their applications in phytoremediation
<130> vMAcp263/113
<160> 22
<170> PatentIn version 3.1
<210> 1 <211> 23
   <212> DNA
   <213> Artificial sequence
<220>
   <223> amorce PCR Rev6HMA4
<400> 1
   gaccaatatg ttgatgtcga tcc 23
<210> 2
   <211> 22
   <212> DNA
   <213> Artificial sequence
<220>
   <223> amorce PCR Rev5HMA4
<400> 2
   ggctttggca agaatcggat ag 22
<210> 3
   <211> 25
   <212> DNA
   <213> Artificial sequence
<220>
   <223> amorce PCR Rev4HMA4
<400> 3
   gcggcaactg ctgccacggc gagcc 25
<210> 4
   <211> 27
   <212> DNA
   <213> Artificial sequence
<220>
   <223> amorce PCR Rev3HMA4
<400> 4
   cttcttcact ttctttttct cttcttc 27
<210> 5
   <211> 23
   <212> DNA
   <213> Artificial sequence
<220>
   <223> amorce PCR Rev2HMA4
<400> 5
   gtagcaaaag gaagaagccg atg 23
<210> 6
   <211> 25
   <212> DNA
   <213> Artificial sequence
<220>
   <223> amorce PCR Rev1HMA4
<400> 6
   ctggtttggt gcgatcagat aaagg 25
<210> 7
   <211> 24
   <212> DNA
   <213> Artificial sequence
<220>
   <223> amorce PCR 1HMA4
<400> 7
   cacttctctc aacctttatc tgat 24
<210> 8
   <211> 24
   <212> DNA
   <213> Artificial sequence
<220>
   <223> amorce PCR Rev14HMA4
<400> 8
   gttattcaat caatctccat caag 24
<210> 9
   <211> 40
   <212> DNA
   <213> Artificial sequence
<220>
   <223> amorce PCR TopoFv1For
<400> 9
   gccgcccggg atccggtacc actagtgaat tcgtcgacga 40
<210> 10
   <211> 35
   <212> DNA
   <213> Artificial sequence
<220>
   <223> amorce PCR HMA4For230
<400> 10
   cgcagcttgc tttactgggt atcaagtgtt gaaag 35
<210> 11
   <211> 31
   <212> DNA
   <213> Artificial sequence
<220>
   <223> amorce PCR Rev2bisHMA4
<400> 11
   catctaaaac attccctagc tgttcttgag c 31
<210> 12
   <211> 36
   <212> DNA
   <213> Artificial sequence
<220>
   <223> amorce PCR Actin8F
<400> 12
   gtggtcgtac aaccggtatt gtgttggact ctggtg 36
<210> 13
   <211> 41
   <212> DNA
   <213> Artificial sequence
<220>
   <223> amorce PCR Actin8R
<400> 13
   acgctgtaac cggaaagttt ctcacatagt gcacaaatga c 41
<210> 14
   <211> 21
   <212> DNA
   <213> Artificial sequence
<220>
   <223> amorce PCR Rev18HMA4
<400> 14
   gccttttgtg gagctaagct c 21
<210> 15
   <211> 25
   <212> DNA
   <213> Artificial sequence
<220>
   <223> amorce PCR 10HMA4
<400> 15
   atggaggcag cagcagttgt gttcc 25
<210> 16
   <211> 24
   <212> DNA
   <213> Artificial sequence
<220>
   <223> amorce PCR Rev8HMA4
<400> 16
   gggacaacca ctgactaaca caac 24
<210> 17
   <211> 39
   <212> DNA
   <213> Artificial sequence
<220>
   <223> amorce PCR 22HMA4
<400> 17
   gagagtgttg gagactgcaa gtctggtcat tgccagaag 39
<210> 18
   <211> 54
   <212> DNA
   <213> Artificial sequence
<220>
   <223> amorce PCR delta HisStopHMA4
<400> 18
   aaggaaaaaa gcggccgcaa aaggaaaatt agctgcataa ctcttttgca taac 54
<210> 19
   <211> 45
   <212> DNA
   <213> Artificial sequence
<220>
   <223> amorce PCR HMA4D401Afor
<400> 19
   gatcaagatt gttgctttcg ctaaaactgg gactattaca agagg 45
<210> 20
   <211> 45
   <212> DNA
   <213> Artificial sequence
<220>
   <223> amorce PCR HMA4D401rev
<400> 20
   cctcttgtaa tagtcccagt tttagcgaaa gcaacaatct tgatc 45
<210> 21
   <211> 26
   <212> DNA
   <213> Artificial sequence
<220>
   <223> amorce PCR 9HMA4
<400> 21
   ccattaaaag gcctaggatc gacatc 26
<210> 22
   <211> 41
   <212> DNA
   <213> Artificial sequence
<220>
   <223> amorce PCR TopoFV1Rev
<400> 22
   tcgtcgacga attcactagt ggtaccggat cccgggcggc c 41